(19) Europäisches Patentamt
European Patent Office
Office européen des brevets

(11) EP 3 103 485 A1

(12) **EUROPEAN PATENT APPLICATION**

(43) Date of publication:
**14.12.2016 Bulletin 2016/50**

(21) Application number: **15305896.1**

(22) Date of filing: **11.06.2015**

(51) Int Cl.:
***A61L 27/48*** *(2006.01)* ***A61L 27/52*** *(2006.01)*
***A61L 27/54*** *(2006.01)* ***A61L 31/12*** *(2006.01)*
***A61L 31/14*** *(2006.01)* ***A61L 31/16*** *(2006.01)*
***A61K 47/10*** *(2006.01)* ***A61K 47/14*** *(2006.01)*
***A61K 47/36*** *(2006.01)* ***A61K 9/107*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **COMMISSARIAT A L'ENERGIE ATOMIQUE ET AUX ENERGIES ALTERNATIVES**
  **75015 Paris (FR)**
- **Centre National de la Recherche Scientifique (C.N.R.S.)**
  **75016 Paris (FR)**
- **Université Grenoble Alpes**
  **38400 Saint-Martin-d'Hères (FR)**

(72) Inventors:
- **TEXIER-NOGUES, Isabelle**
  **38000 GRENOBLE (FR)**
- **AUZELY-VELTY, Rachel**
  **38750 LE GUA (FR)**
- **GIDROL, Xavier**
  **06570 SAINT PAUL DE VENCE (FR)**
- **NAVARRO Y GARCIA, Fabrice**
  **38600 FONTAINE (FR)**
- **RACINE, Lisa**
  **38000 GRENOBLE (FR)**
- **TEZGEL, Ozgul**
  **38000 GRENOBLE (FR)**

(74) Representative: **Lavoix**
**2, place d'Estienne d'Orves**
**75441 Paris Cedex 09 (FR)**

(54) **MATERIAL COMPRISING A POLYMER CAPABLE OF FORMING A HYDROGEL AND NANOPARTICLES**

(57) The present invention concerns a material comprising a polymer capable of forming a hydrogel and nanoparticles comprising a solubilising lipid comprising at least one fatty acid glyceride, an amphiphilic lipid, and a co-surfactant comprising at least one poly(ethylene oxide) chain or a chitosan chain, the process for the preparation thereof and the uses thereof.

EP 3 103 485 A1

## Description

The present invention concerns a material comprising a polymer capable of forming a hydrogel and nanoparticles, the process for the preparation thereof and the uses thereof, in particular for tissue engineering, cell culture, dressing-design applications and for the delivery of therapeutic agents.

A hydrogel is a physically or chemically cross-linked natural or synthetic polymeric network, which has the capacity to absorb a large fraction of water (up to thousands of times their dry weight), although typically of limited solubility in this media. The water retention capacity of hydrogels arises mainly from the presence of hydrophilic groups (amido, amino, carboxyl, hydroxyl, etc.) in the polymer chains and the degree of swelling can be modulated by the polymer composition and the density and nature of cross-links in the gel matrix. The water content of a hydrogel determines its unique physicochemical characteristics that can resemble those of living tissues more than any other class of synthetic biomaterials. These are largely attributed to their high water content, their soft and elastic consistency, and low interfacial tension when in contact with water or biological fluids.

The development of alternative hydrogels the physicochemical features of which (for example its mechanical properties and/or its hydrophily) and/or whose behaviour in their environment (for example having a structure adequate to cell culture and proliferation and/or presenting a higher resistance to degradation) might be tailored depending on the desired application, while maintaining the advantageous properties of hydrogel is needed.

Besides, patent application WO 2010/018223 describes the use of a formulation in the form of a nanoemulsion comprising an aqueous phase and at least one dispersed phase comprising an amphiphilic lipid, a solubilising lipid, a therapeutic agent and a co-surfactant comprising at least one chain composed of alkylene oxide repeat units to deliver an amphiphilic or lipophilic therapeutic agent.

This formulation can be in a gel form by using a specific amount of dispersed phase, as described in application WO 2011/101602, or by covalently linking the nanoparticles of the emulsion, as described in application WO 2013/144369. However, the porosity of these gels is expected to be of the same order of magnitude as the nanoparticles size, i.e. in the nanometric size. This is not well suited for cell colonization, like required for cell culture, tissue engineering or advanced dressing applications, wherein porosity in the micrometric size is required. The development of alternative materials to these gels is therefore required for these applications.

For this purpose, the present invention provides a material comprising a polymer capable of forming a hydrogel and nanoparticles.

### [Material]

Accordingly, a first object of the invention is the material comprising:

- a polymer capable of forming a hydrogel, and
- nanoparticles comprising:
- a solubilising lipid comprising at least one fatty acid glyceride,
- an amphiphilic lipid, and
- a co-surfactant comprising at least one poly(ethylene oxide) chain or a chitosan chain.

Generally, the weight of polymer capable of forming a hydrogel in the material compared to the weight of the nanoparticles (i.e. the total weight of the nanoparticles components) is from 0.05/1 to 1000/1, preferably from 0.1/1 to 500/1, most preferably from 2/1 to 500/1.

- Components of the material according to the invention
- Polymer capable of forming a hydrogel

The polymer capable of forming a hydrogel can be of synthetic or natural origin (i.e. biopolymers), or both (copolymers mixing the two).

Generally, the synthetic (co)polymers capable of forming a hydrogel are obtained by polymerizing monomers chosen from hydroxyethyl methacrylate (HEMA), hydroxyethoxyethyl methacrylate (HEEMA), hydroxydiethoxyethyl methacrylate (HDEEMA), methoxyethyl methacrylate (MEMA), methoxyethoxyethyl methacrylate (MEEMA), methoxydiethoxyethyl methacrylate (MDEEMA), ethylene glycol dimethacrylate (EGDMA), N-vinyl-2-pyrrolidone (NVP), N-isopropyl acrylamide (NIPAAm), vinyl acetate (VAc), acrylic acid (AA), methacrylic acid (MAA), N-(2-hydroxypropyl)-methacrlamide (HPMA), ethylene glycol (EG), poly (ethylene glycol) acrylate (PEGA), poly (ethylene glycol) methacrylate (PEGMA), poly (ethylene glycol) diacrylate (PEGDA), poly (ethylene glycol) dimethacrylate (PEGDMA), lactic acid and glycolic acid and a mixtures thereof, preferably from ethylene glycol, hydroxyethyl methacrylate, N-isopropyl acrylamide and a mixtures thereof.

Generally, the biopolymer capable of forming a hydrogel is:

- a polysaccharide, typically chosen among chondroitin sulfate, hyaluronic acid, hyaluronan, chitosan, cellulose and its water-soluble derivatives such as carboxymethylcellulose (CMC), methylcellulose, hydroxyethylcellulose, hydroxypropylcellulose, alginate, glycosaminoglycannes (GAGs), such as heparan sulfate or chondroitin sulfate, preferably chosen among chitosan, carboxymethylcellulose, hydroxyethylcellulose, cellulose, hyaluronic acid, and hyaluronan, more preferably chitosan and carboxymethylcellulose,
- a protein, typically chosen among collagen, gelatin, elastin and fibroin (silk), preferably collagen and gelatin, more preferably collagen.

The polymer capable of forming a hydrogel can be a homopolymer (for instance: collagen or chitosan) or a copolymer (for instance CMC-PEG and chitosan-PEG).

The preferred polymers are chitosan, chitosan-PEG copolymers, CMC, CMC-PEG copolymers, collagen and hyaluronic acid.

Biocompatible and/or biodegradable polymers, such as chitosan, collagen, carboxymethylcellulose and hyaluronic acid, are preferred, as the material obtained at the end of the process will then also be biocompatible and/or biodegradable. Chitosan advantageously displays fibrin-mediated targeting and adhesion to mucus layers. Hyaluronic acid advantageously displays CD44-mediated targeting properties (targeting macrophages).

Chitosan, a partially deacetylated derivative of chitin, obtained from the shells of crabs and shrimps, is a cationic polymer whose properties can be tuned with the pH. It is a candidate of choice for biomedical and biological applications since it presents high biocompatibility and biodegradability. From natural origin, chitosan is generally soluble in slightly acidic medium (pH 4, acetylation degree $<0.5$) which allows its chemical modification in mild conditions. Chitosan is known to improve the kinetics of wound healing by stimulating the immune response and tissue reconstruction, by preventing bacterial infections, and by its high exudate absorbing properties. It is as well a good substrate for cell culture and stimulates cell growth. All these properties make chitosan a good candidate material for a wide range of applications, including wound healing, regenerative medicine (bone reconstruction in particular), and the design of drug delivery devices (implants, solutions, hydrogels, patches...).

Hyaluronic acid is a high molar mass (6-7000 kg/mol) linear glycosaminoglycan found in soft tissues such as connective, epithelial, and neuronal tissues and synovial fluid as well. This polysaccharide can be obtained with a high degree of purity (pharmaceutical grade) on a large scale by bacterial fermentation, which makes it very attractive for designing soft materials in the biomedical field. Hyaluronic acid plays an important role in many biological processes including cell proliferation, cell differentiation, morphogenesis, inflammation, and wound repair. Due to its unique viscoelastic and biological properties, hyaluronic acid finds itself in many biomedical applications such as ophthalmological surgery, treatment of osteoarthritis of the knee, prevention of post-surgical adhesion... In addition, hyaluronic acid has excellent potential for constructing synthetic Extra Cellular Matrix analogs

Water-soluble derivatives cellulose such as carboxymethylcellulose, methylcellulose, hydroxyethylcellulose, hydroxypropylcellulose are used in the field of food, pharmaceuticals and cosmetics for its biocompatibility and biodegradability applications. Carboxymethylcellulose (CMC) is the most important cellulose derivatives. It is obtained from the esterification of hydroxyl groups at the 2, 3 and 6 positions of the glucose units of cellulose.This negatively charged polysaccharide is highly water soluble, allowing chemical modification in soft conditions. CMC hydrogels have been widely used for medical applications (drug delivery, implant for regenerative medicine...) because of their excellent properties such as high water content and good biodegradation, as well as their relatively low cost.

Collagens are stable, harmless and well tolerated topically and are widely used on an industrial scale as components of formulations, medical devices and cosmetics intended for topical dermatological application. The synthesis of collagen is amplified during the proliferative phase of dermal tissue repair stimulating the proliferation of granulation tissue and accelerating the repair tissue injury.

Collagens are synthesized by fibroblasts and are the major proteins entering the composition of the Extra-Cellular Matrix (ECM), the major structural proteins of any organ, and the most abundant proteins in the body. Collagen exhibits high biocompatibility, biodegradability, and weak antigenicity, which makes it a biomaterial of choice for nanomedicine applications (tissue engineering, 3D cell culture materials, dressings...).

The advantages of collagen in tissue engineering, 3D cell culture and dressing-design applications lay in its fiber-organized structure and the high tensile strength of the fibers. Moreover, it is a material convenient for processing, since it can be prepared and stored in different forms, such as elastic solid, films, gels, sprays, powder... When hydrated or in contact with biological fluids, collagen is a hydrogel material. For storage or easier manipulation, the gel can be dehydrated by lyophilization process. Moreover, the strength of the fibers and strain-resistance of the material can be tuned according to the choice of collagen, and improved by chemical cross-linking.

Disadvantages of collagen include high cost and variability of the material (extracts of animal origin), as well as its important hydrophilicity which induces high swelling and rapid release of the material content, as well as fast

enzymatic degradation especially in the presence of metalloproteases (MMPs). In this regard, MMPs are over-expressed in an important number of pathologies, especially in wound beads.

There are different types of collagens, distincts from the chemical and structural point of view, including the native collagen of type I, which represents 70% of the collagen of the dermal matrix and is essential in tissue reconstruction.

In one embodiment, the polymer capable of forming a hydrogel is undenaturated collagen, i.e. collagen of type I. Preferably, the collagen is extracted from horse tendon.

• Nanoparticles

The material according to the invention comprises nanoparticles comprising:

- a solubilising lipid comprising at least one fatty acid glyceride,
- an amphiphilic lipid, and
- a co-surfactant comprising at least one poly(ethylene oxide) chain or a chitosan chain.

The nanoparticles can comprise additional components, in particular chosen from an oil, a therapeutic agent, an imaging agent, a cationic surfactant, a functionalizable surfactant, a thickening agent, other additives and a mixture thereof.

◦ Amphiphilic lipid

The nanoparticles of the material comprises at least one amphiphilic lipid.

The amphiphilic lipids comprise a hydrophilic part and a lipophilic part. They are generally selected from among compounds whose lipophilic part comprises a saturated or unsaturated, linear or branched chain having 8 to 30 carbon atoms. They can be selected from among phospholipids, cholesterols, lysolipids, sphingomyelins, tocopherols, glucolipids, stearylamines, cardiolipins of natural or synthetic origin; molecules composed of a fatty acid coupled to a hydrophilic group via an ether or ester function such as the esters of sorbitan e.g. sorbitan monooleate and monolaurate marketed under the trade name Span® by Sigma; polymerised lipids; lipids conjugated to short chains of polyethylene oxide (PEG) such as the non-ionic surfactants sold under the trade names Tween® by ICI Americas, Inc. and Triton® by Union Carbide Corp.; sugar esters such as mono- and di-laurate, mono- and di-palmitate, sucrose mono- and distearate; the said surfactants can be used alone or in a mixture.

Phospholipids are the preferred amphiphilic lipids.

Lecithin is a particularly preferred amphiphilic lipid.

◦ Solubilising lipid

The nanoparticles of the material also comprise a solubilising lipid comprising at least one fatty acid glyceride.

The solubilising lipid is a lipid having sufficient affinity for the amphiphilic lipid to ease its solubilization. It may be an oil or wax. Preferably the solubilising lipid is solid at room temperature (20°C), but liquid at body temperature (37°C).)

If the amphiphilic lipid is a phospholipid, the solubilising lipid may in particular be a derivative of glycerol and in particular of glycerides obtained by esterification of glycerol with fatty acids.

The preferred solubilising lipid, in particular for the phospholipids, is a mixture of glycerides of fatty acids in particular of saturated fatty acids, and particularly saturated fatty acids having 8 to 18 carbon atoms, more preferably 12 to 18 carbon atoms.

Preferably, the solubilising lipid is a mixture of glycerides of saturated fatty acids comprising at least 10 % by weight of C12 fatty acids, at least 5 % by weight of C14 fatty acids, at least 5 % by weight of C16 fatty acids and at least 5 % by weight of C18 fatty acids.

Preferably, the solubilising lipid is a mixture of glycerides of fatty acids comprising 0 % to 20 % by weight of C8 fatty acids, 0 % to 20 % by weight of C10 fatty acids, 10 % to 70 % by weight of C12 fatty acids, 5 % to 30 % by weight of C14 fatty acids, 5 % to 30 % by weight of C16 fatty acids and 5 % to 30 % by weight of C18 fatty acids.

Particularly preferred are mixtures of semi-synthetic glycerides solid at room temperature sold under the trade name Suppocire®NC by Gattefossé and approved for injection in man. N-type Suppocire® are obtained by direct esterification of fatty acids and glycerol. They are semi-synthetic glycerides of saturated C8 to C18 fatty acids of which the quali-quantitative composition is given in the Table below.

Fatty acid composition of Suppocire® NC by Gattefossé

| Chain length | [weight %] |
|---|---|
| C8 | 0.1 to 0.9 |
| C10 | 0.1 to 0.9 |
| C12 | 25 to 50 |
| C14 | 10 to 24.9 |
| C16 | 10 to 24.9 |
| C18 | 10 to 24.9 |

o Co-surfactant

The nanoparticles of the material comprise a co-surfactant which allows stabilization of the nanoparticles.

The co-surfactant is generally a water-soluble surfactant. The co-surfactant comprises at least one poly(ethylene oxide) chain or at least one chitosan chain. These chains are hydrophilic.

The co-surfactant generally also comprises a lipophilic chain, usually a hydrocarbon chain, such as a C10-C24 hydrocarbon chain, for example a C10-C24 saturated or unsaturated linear hydrocarbon chain, in particular a C12 or C17 unsaturated linear hydrocarbon chain, for example derives from lauric or stearic acid.

In one embodiment, the co-surfactant comprises at least one poly(ethylene oxide) chain. Preferably, the number of ethylene oxide units in the poly(ethylene oxide) chain varies between 2 and 500, such as from 25 to 400.

As examples of co-surfactants particular mention can be made of the conjugated compounds polyethylene glycol/phosphatidyl-ethanolamine (PEG-PE), the ethers of fatty acid and of polyethylene glycol such as the products sold under the trade names Brij® (for example Brij® 35, 58, 78 or 98) by ICI Americas Inc., the esters of fatty acid and of polyethylene glycol such as the products sold under the trade names Myrj® by ICI Americas Inc. (for example Myrj® 45, 52, 53 ou 59) and the block copolymers of ethylene oxide and propylene oxide such as the products sold under the trade names Pluronic® by BASF AG (for example Pluronic® F68, F127, L64, L61, 10R4, 17R2, 17R4, 25R2 or 25R4) or the products sold under the trade name Synperonic® by Unichema Chemie BV (for example Synperonic® PE/F68, PE/L61 or PE/L64). Preferably, the co-surfactant is an ester of fatty acid (preferably a C12-C24 fatty acid, more preferably stearic acid) and of polyethylene glycol.

In one embodiment, the co-surfactant comprises at least one chitosan chain. Preferably, the chitosan chain displays a molecular weight comprised between 1,000 and 1,000,000 Da, such as from 5,000 to 500,000 Da, more preferably between 15,000 to 300,000 Da. The chitosan chain may be acetylated. Preferably, the co-surfactant is substituted with hydrocarbon chains, such as hydrocarbon chains as described above, with a degree of substitution preferably from 0.005 to 0.1, more preferably from 0.01 to 0.05. This co-surfactant may be obtained by reacting chitosan with a hydrocarbon chain carrying an aldehyde group, preferably a fatty aldehyde such as lauryl aldehyde in the presence of a reducing agent such as sodium cyanohydroborate.

In one embodiment, the co-surfactant comprises a therapeutic agent as defined below. In this embodiment, the process allows preparing a material the surface of the nanoparticles of which has already been grafted with a therapeutic agent.

◦ Oil

In an embodiment, the nanoparticles of the material comprise one or more other oils.

The oils used preferably have a hydrophilic-lipophilic balance (HLB) of less than 8 and more preferably of between 3 and 6. Advantageously the oils are used without chemical or physical modification prior to the forming of the nanoparticles.

The oils are generally selected from among biocompatible oils, and in particular from among oils of natural origin (vegetable or animal) or synthetic. Among these oils particular mention can be made of oils of natural vegetable origin amongst which are included soybean, flax, palm, groundnut, olive, grape seed and sunflower seed oil; synthetic oils which particularly include triglycerides, diglycerides and monoglycerides. These oils may be first press, refined or inter-esterified oils.

The preferred oils are soybean oil and flax oil.

◦ Therapeutic agent

In a preferred embodiment, the nanoparticles of the material comprise a therapeutic agent.

Thus, in this embodiment, at the end of the process according to the invention, a material comprising a polymer capable of forming a hydrogel and nanoparticles loaded with a therapeutic agent can be obtained without disturbing the structure of the polymer capable of forming a hydrogel matrix, especially its porosity.

The therapeutic agent may be pharmaceutical active ingredients or biological agents such as DNA, proteins, peptides or antibodies or agents useful for physical therapy such as compounds useful for heat therapy, compounds releasing singlet oxygen when excited by light and used for phototherapy, and radioactive agents.

The type of therapeutic agent is not particularly limited. The mild conditions of the process allow using therapeutic agents which are degraded at high temperature.

Among the pharmaceutical active ingredients of interest as therapeutic agents, particular mention can be made of the agents used in the treatment of AIDS, the agents used in the treatment of heart disease, analgesics anaesthetics, anorexigenics, anthelmintics, antiallergics, antianginals, antiarrhythmics, anticholinergics, anticoagulants, antidepressants, antidiabetics, antidiuretics, antiemetics, anticonvulsants, antifungals, antihistaminics, antihypertensives, anti-inflammatories, antimicrobial agents, antimigraines, antimuscarinics, antimycobacterials, chemotherapeutic agents, anticancer drugs including antiparkinsons, antithyroid drugs, antivirals, astringents, blocking agents, blood products, blood substitutes, cardiac inotropic agents, cardiovascular agents, central nervous system agents, chelators, chemotherapy agents, hematopoietic growth factors, cell adhesion factors, cell migration factor, corticosteroids, antitussives, dermatological agents, diuretics, dopaminergic drugs, elastase inhibitors, endocrine agents, ergot alkaloids, expectorants, gastro-intestinal agents, genitourinary agents, growth hormone triggering factor, growth hormones, hematologic agents, hematopoietic agents, hemostatics, hormones, vitamins, immunosuppressives, interleukins, analogues of interleukins, lipid regulating agents, gonadoliberin, muscle relaxants, narcotic antagonists, nutrients, nutritive agents, oncology therapy, organic nitrates, vagomimetics, prostaglandins, antibiotics, renal agents, respiratory agents, sedatives, sexual hormones, stimulants, sympathomimetics, systemic anti-infectious agents, tacrolimus, thrombolytic agents, thyroid agents, treatments for attention disorders, vasodilators, xanthines, cholesterol-reducing agents.

Particularly interesting, especially for 3D cell culture applications, are hormones, vitamins, growth factors, cell adhesion factors, cell migration factors, chemotherapeutic agents, antibiotics, antimicrobial agents, saccharides, nucleotide sequences, such as DNA, mRNA or nucleotide sequences able to modulate endogenous mechanisms of RNA interference for example SiRNA.

Particularly interesting, especially for dressing, implant or implant coating applications, are chemical compounds capable of promoting wound healing, growth factors, antibiotics, antimicrobial agents, saccharides, and nucleotide sequences, such as DNA, mRNA or nucleotide sequences able to modulate endogenous mechanisms of RNA interference for example SiRNA.

Particularly interesting, especially for vaccine or antibodies production applications, are antigens or nucleic acid molecules encoding one or several protein antigens. The antigen is in particular such as described p. 6, l. 13 to p. 9, l. 14 of PCT application WO2014/131809. The antigen is preferably a proteic or a peptidic antigen.

The therapeutic agents can be either encapsulated in the core of the nanoparticles, loaded inside the shell, or grafted onto the surface of the nanoparticles. In one embodiment, the surface of the nanoparticles of the material has been grafted (either by covalent or electrostatic binding) with the therapeutic agent.

The therapeutic agent can be formulated directly in its active form or in prodrug form.

Also, the nanoparticles may comprise several therapeutic agents.

The material can also comprise different types of nanoparticles, each type of nanoparticle comprising a therapeutic agent of different nature. In an embodiment, the material comprises different types of nanoparticles, each type of nanoparticle comprising a growth factor of different nature.

◦ Imaging agent

In one embodiment, the nanoparticles of the material comprise an imaging agent which advantageously allows visualisation of the distribution of the nanoparticles.

The imaging agent may be used in particular in imaging of the following type:

- Positron Emission Tomography (PET) (the imaging agent possibly being a compound comprising a radionuclide such as $^{18}F$, $^{11}C$, a chelate of metal cations $^{68}Ga$, $^{64}Cu$),
- Single Photon Emission Computed Tomography (SPECT) (the imaging agent possibly being a compound comprising a radionuclide for example $^{123}I$, or a chelate of $^{99m}Tc$ or $^{111}In$),
- Magnetic Resonance Imaging (MRI) (the imaging agent possibly being a chelate of gadolinium or a magnetic nanocrystal such as a nanocrystal of iron oxide, manganese oxide of iron-platinum FePt),

- Optical imaging or X-ray imaging (the imaging agent possibly being a lipophilic fluorophore or a contrast agent, for example an iodine molecule such as iopamidol, amidotrizoate, or gold nanoparticles).

Preferably the imaging agent is a lipophilic fluorophore allowing the performing of optical imaging.
The type of lipophilic fluorophore(s) used is not critical provided that they are compatible with *in vivo* imaging (i.e. they are biocompatible and non-toxic). Preferably the fluorophores used as imaging agent absorb and emit in the visible or near infrared. For non-invasive imaging, in a tissue or living body (animal, man) the preferred fluorophores absorb and emit in the near infrared. For best passing through the tissues of the excitation light and the light emitted by the fluorophore, the most suitable fluorophores are those absorbing and emitting in the near infrared i.e. a wavelength of between 640 and 900 nm.
As lipophilic fluorophore it is possible to cite the compounds described in Chapter 13 ("Probes for Lipids and Membranes") in the InVitrogen catalogue. More specifically, as fluorophore mention can particularly be made of indocyanine green (ICG), the analogues of fatty acids and phospholipids functionalised by a fluorescent group such as the fluorescent products sold under the trade names Bodipy (R) for example Bodipy (R) 665/676 (Ex/Em.); the lipophilic derivatives of carbocyanines such as 1,1'--dioctadecyl-3,3,3',3'-tetramethylindodicarbocyanine perchlorate (DiD) sold for example under the reference D-307, 3,3'-dihexadecyloxacarbocyanine perchlorate (DiO) sold for example under the reference D1125, 1,1'-dihexadecyl-3,3,3',3'-tetramethylindocarbocyanine perchlorate (Dil) sold for example under the reference D384; fluorescent probes derived from sphingolipids, from steroids or from lipopolysaccharides such as the products sold under the trade names BODIPY ® TR ceramids, BODIPY ® FL C5-lactosylceramide, BODIPY ® FL C5-ganglioside, BODIPY ® FL cerebrosides; the amphiphilic derivatives of cyanines, of rhodamines, of fluoresceins or coumarins such as octadecyl rhodamine B, octadecyl ester of fluorescein and 4-heptadecyl-7-hydroxycoumarin; and diphenylhexatriène (DPH) and derivatives thereof; all these products being sold by Invitrogen.
According to a preferred embodiment, the fluorophore is indocyanine green, 1,1'-dioctadecyl-3,3,3',3'-tetramethylindodicarbocyanine perchlorate, 3,3'-dihexadecyloxacarbocyanine perchlorate, or 1,1'-dihexadecyl-3,3,3',3'-tetramethylindocarbocyanine perchlorate

o Cationic surfactant

In one embodiment, the nanoparticles of the material comprise a cationic surfactant, such as a cationic surfactant described on pages 6 to 13 of application WO 2014/032953.
Preferably, the cationic surfactant is selected from among:

- *N*[1-(2,3-dioleyloxy)propyl]-*N,N,N*-trimethylammonium (DOTMA),
- 1,2-dioleyl-3-trimethylamonium-propane (DOTAP),
- *N*-(2-hydroxyethyl)-*N*,*N*-dimethyl-2,3-bis(tetradecyloxy-1-propananium) (DMRIE),
- 1-[2-(oleoyloxy)ethyl]-2-oleyl-3-(2-hydroxyethyl)imidazolinium (DOTIM), and
- dioctadecylamidoglycylspermine (DOGS) (in protonated form).

The cationic surfactant is most preferably 1,2-dioleyl-3-trimethylamonium-propane (DOTAP).
This cationic surfactant allows the loading of nucleotide sequences through electrostatic interactions; said nucleotide sequences being generally DNA sequences, mRNA or nucleotide sequences able to modulate endogenous mechanisms of RNA interference to the nanoparticles, in particular a single or double strand nucleotide sequence, comprising fewer than 200 bases for a single strand nucleotide sequence, or fewer than 200 base pairs for a double strand nucleotide sequence, that is able to modulate endogenous mechanisms of interfering RNA (ribonucleic acid), such as:

- a short or small interfering RNA - siRNA,
- a locked nucleic acid - LNA,
- a synthetic microRNA mimic called MicroRNA or miRNA,

In particular those described on pages 38 to 39 of application WO 2014/032953.
The said nucleotide sequence is preferably a siRNA or a microRNA.
Preferably, when the nanoparticles comprise a cationic surfactant, they also comprise a helper lipid which is able to facilitate cytosolic release by destabilising the endosomal membrane, such as dioleylphosphatidylethanolamine (DOPE).
In one embodiment, when the nanoparticles comprise a cationic surfactant, the co-surfactant comprises a chain comprising at least 25 ethylene oxide units, as described on page 18 of application WO 2014/032953.
In another embodiment, when the nanoparticles comprise a cationic surfactant, the co-surfactant comprises at

least one chitosan chain as described above.

Preferably, when the nanoparticles comprise a cationic surfactant, the proportions of the different components of the nanoparticles are preferably as described in application WO 2014/032953.

o Anionic surfactant

In one embodiment, the nanoparticles of the material comprise an anionic surfactant, such as cetyl phosphate.

Preferably, the nanoparticles of the material comprise either an anionic surfactant or a cationic surfactant, but not both.

◦ Functionalizable surfactant

In an embodiment, the nanoparticles of the material comprise a functionalizable surfactant. In the present application, a functionalizable surfactant is a surfactant comprising at least a functionalizable group on which a chemical compound can be grafted. Generally, the chemical compound is a therapeutic agent, preferably as defined above, more preferably an antigen, or a nucleic acid an antigen. This functionalizable surfactant allows grafting a therapeutic agent onto the nanoparticles, preferably via the formation of a covalent bond.

Typically, the functionalizable surfactant is a surfactant of formula (LI) :

$$L_1\text{-}X_1\text{-}H_1\text{-}Y_1\text{-}G_1 \qquad \text{(LI)},$$

wherein :

- $L_1$ represents a lipophilic group,
- $X_1$ and $Y_1$ independantly represent a binding group,
- $H_1$ represents an hydrophilic group comprising a polyalkoxylated chain,
- $G_1$ is a group that can react with the therapeutic agent,

preferably such as described in PCT application WO 2014/131809 (in particular on p. 11, l. 8 to p. 13, l. 4).

Preferably, the functionalizable surfactant is a surfactant of formula (CI), (CII), (CIII) or (CIV):

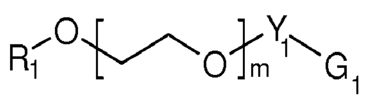
(CI) ,

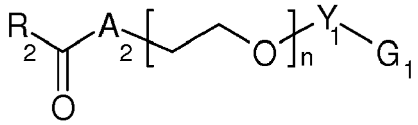
(CII)

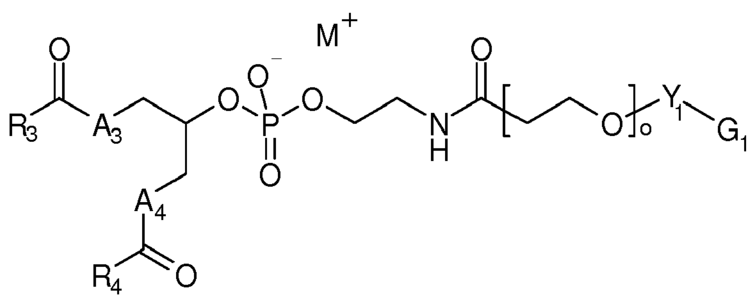
(CIII) ,

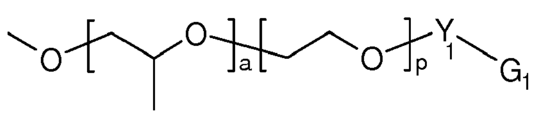
(CIV)

wherein :

- $R_1$, $R_2$, $R_3$ and $R_4$ independantly represent a linear hydrocarbon chain comprising from 7 to 23 carbon atoms,
- $A_1$, $A_2$, $A_3$ and $A_4$ represent O or NH,
- m, n, o and p independantly represent integer from 3 to 500, preferably from 20 to 200, and
- a represents an integer from 20 to 120,
- M represents H or a cation,
- $Y_1$ represents a binding group,
- $G_1$ is a group that can react with the therapeutic agent.

The skilled person knows which functionalizable group should be chosen depending on the nature of the chemical compound to be grafted. For example, when the chemical compound comprises a thiol group, a surfactant comprising, as functionalizable group (i.e. as $G_1$ group), a maleimide group is appropriate as these functions can react with each other.

For example, the functionalizable surfactant has the formula (LI'):

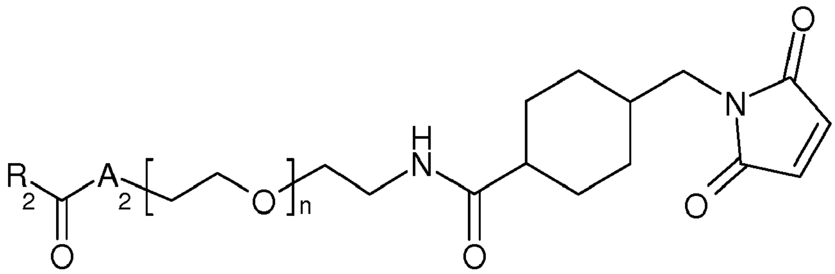

(LI')

wherein $R_2$, $A_2$ and n as are defined above.

◦ Thickening agent

In an embodiment, the nanoparticles of the material comprise a such as glycerol, a saccharide, oligosaccharide or polysaccharide, a gum or a protein, preferably glycerol. o Other additives

The nanoparticles may further contain other additives such as dyes, stabilisers, pH adjusting agent and/or preserving agents.

Preferably, the material is free from metal, or from inorganic components.

Preferably, the material is free from nanotubes, in particular from carbon nanotubes.

Preferably, the material is free from cyclodextrins.

• Other components of the material

In one embodiment, the material comprises a therapeutic agent, such as one of the therapeutic agents described above, preferably a hydrophilic agent. This therapeutic agent is not loaded in the nanoparticles of the material, contrarily to the therapeutic agent described above, but is located within the polymer matrix. Preferably, in this embodiment, the nanoparticles also comprise a therapeutic agent, but of different nature.

• Form of the material according to the invention

In the material according to the invention, the nanoparticles are generally homogeneously distributed.

In the material, the nanoparticles generally present a spherical shape. The mean diameter of the nanoparticles of the material is generally greater than 10 nm and smaller than 500 nm, preferably between 20 and 400 nm, most preferably between 30 and 300 nm.

The material can be supported on a substrate.

◦ Hydrogel form

In an embodiment, the material is in a hydrogel form, i.e. the material comprises an aqueous phase, such as water or a buffer solution. Generally, the material comprises more than 50%wt of aqueous phase.

Advantageously, the shearing properties of the material in a hydrogel form are close to the one of the polymer capable of forming a hydrogel that it comprises. The shearing properties can be assessed by the elastic modulus G'. G' can be determined using a rheometer, for example an AR2000Ex rheometer (TA Instruments, Inc.). Generally, G' of the material is from 70 to 130%, preferably from 75 to 125%, more preferably from 85 to 115% of the elastic modulus of the hydrogel formed by the polymer capable of forming a hydrogel (It being understood that the polymer capable of forming a hydrogel is the same than the polymer capable of forming a hydrogel comprised in the material, and that the hydrogel formed by the polymer capable of forming a hydrogel is free from nanoparticles). This could be due to the nature of the nanoparticles included in the material, which could be easily deformable under shearing stress. On the contrary, the compression properties as assessed by the Young modulus E of the material in a hydrogel form are generally impacted by the presence of the nanoparticles.

◦ Dry form

In an embodiment, the material is in a dry form, for example a film form, a powder form, or in a solid form.
In an embodiment, the material comprises less than 25%wt of water. The water proportion can be determined using a Karl Fisher apparatus.
In an embodiment, the material is a porous material, in particular when the polymer capable of forming a hydrogel is collagen. The material is then in the form of a sponge.
When the polymer capable of forming a hydrogel is collagen, the material typically comprises pores the Feret's diameter of which are between 50 and 150 μm. The Feret's diameter can be determined using Scanning Electron Microscopy (SEM) images, for example images obtained from a JEOL JSM 6010LA electron microscope and then using a software to analyze the diameters of the pores, for example the ImageJ® software. A fibrous structure with an average pore size of 50-150 μm is an ideal structure for collagen material for cell growth (C. J. Doillon, C. F. Whyne, S. NBrandwein, F. H. Silver. "Collagen-based wound dressings: control of the pore structure and morphology". J. Biomed. Mater. Res., vol.20, pp.1219-1228, 1986).
Preferably, the swelling ratio (SR) of the material in 1X PBS is close to the one of the polymer capable of forming a hydrogel that it comprises. Generally, the swelling ratio of the material in 1X PBS is from 60 to 100%, preferably from 70 to 100%, more preferably from 75 to 100% of the swelling ratio of the polymer capable of forming a hydrogel (It being understood that the polymer capable of forming a hydrogel is the same than the polymer capable of forming a hydrogel comprised in the material. For example if the material comprises Type I collagen as polymer capable of forming a hydrogel, the swelling ratio of the material is from 60 to 100% of the swelling ratio of Type I collagen). ratio of the material is from 60 to 100% of the swelling ratio of Type I collagen).
The swelling ratio (SR) can be calculated according to the following equation:

$$SR = 100 \times (W_w - W_d) / W_d$$

in which $W_d$ is the weight of material (dry material) and $W_w$ is the material after 24 hours immersion in 1X PBS.
When the polymer capable of forming a hydrogel is collagen, as the nanoparticles are smaller than collagen fibers, the nanoparticles coat the collagen fiber matrix without disturbing the arrangement of the fibers. The nanoparticles are coated on the collagen fibers and are regularly distributed. The collagen structure and its porosity are not modified since the nanoparticles are very small in comparison to collagen fibers. However, the physicochemical properties of the collagen fiber matrix are modified by this coating. Advantageously, the material according to the invention:

- is less hydrophilic than the polymer capable of forming a hydrogel (on its own), and/or
- exhibits a higher resistance to degradation than the polymer capable of forming a hydrogel (on its own) (in particular when in contact with cells),

while maintaining the structure and porosity of the polymer capable of forming a hydrogel.
The material in dry form can be stored before use. It usually has a longer shelf-life then the hydrogel form.

**[Process for the preparation of the material]**

• Alternative methods for forming the material in hydrogel form

The process for the preparation of the material usually comprises the preparation of the material in hydrogel form, and then its drying when a dry form of the material is desired.

The material in hydrogel form according to the invention can be prepared according to two alternative processes.

• Method A

A second object of the present invention is a process for the preparation of a material as described above, comprising the steps consisting in:

A1) providing a nanoemulsion comprising a continuous aqueous phase and nanoparticles comprising an amphiphilic lipid, a solubilising lipid comprising at least one fatty acid glyceride and a co-surfactant comprising at least one poly(ethylene oxide) chain or a chitosan chain,
A2) mixing the nanoemulsion obtained in step A1) with a polymer capable of forming a hydrogel to obtain the material in hydrogel form.

Thus, according to method A, a nanoemulsion comprising a continuous aqueous phase and nanoparticles (which is the dispersed phase of the nanoemulsion) is first provided and then mixed with a polymer capable of forming a hydrogel. The nanoparticles form the dispersed phase of the nanoemulsion. The nanoemulsion of step A1) is generally free from polymer capable of forming a hydrogel.
The nanoemulsion of step A1) is typically obtained by a process comprising the following steps:

A1a) preparing an oily phase comprising the solubilising lipid and the amphiphilic lipid,
A1b) preparing an aqueous phase comprising the co-surfactant;
A1c) dispersing the oily phase in the aqueous phase under the action of sufficient shear to form a nanoemulsion,
A1d) optionally recovering the nanoemulsion thus formed.

• Method B

A third object of the present invention is a process for the preparation of a material as described above, comprising the steps consisting in:

B1) preparing an oily phase comprising a solubilising lipid comprising at least one fatty acid glyceride and an amphiphilic lipid,
B2) preparing an aqueous phase comprising a co-surfactant comprising at least one poly(ethylene oxide) chain or a chitosan chain and a polymer capable of forming a hydrogel;
B3) dispersing the oily phase in the aqueous phase under the action of sufficient shear to obtain the material in hydrogel form.

Thus, according to method B, the polymer capable of forming a hydrogel is present in the aqueous phase before formation of the nanoparticles.

• Steps of methods A and B

• Step A1 a) (method A) or step B1) (method B): Preparation of the oily phase

The preparation of the oily phase comprises the mixing of the oily components of the nanoparticles, i.e. the amphiphilic lipid, the solubilising lipid, the optional oil, the optional imaging agent, the optional therapeutic agent, the optional cationic surfactant, the optional anionic surfactant and the optional helper lipid.
To form a stable nanoemulsion, it is necessary to include in the oily phase at least one amphiphilic lipid as surfactant. The amphiphilic nature of the surfactant ensures the stabilisation of the nanoparticles in the continuous aqueous phase.
Generally, the oily phase comprises 0.01 to 99 % by weight, preferably 1 to 60 % by weight, in particular 5 to 25 % by weight of amphiphilic lipid. The quantity of amphiphilic lipid advantageously contributes towards controlling the size of the nanoparticles.
The aforementioned solubilising lipids allow a stable nanoemulsion to be obtained. Without wishing to be bound by any particular theory, it is assumed that the aforementioned solubilising lipids allow obtaining nanoparticles in the nanoemulsion which have an amorphous core. The core thus obtained has higher internal viscosity without exhibiting any crystallinity. Crystallisation is harmful for the stability of the nanoemulsion since it generally leads to aggregation of the nanoparticles and/or to expelling of the encapsulated molecules outside the nanoparticles. These physical properties promote the physical stability of the nanoemulsion.

The amount of solubilising lipid may vary extensively in relation to the type and amount of amphiphilic lipid contained in the nanoparticles. In general, the oily phase comprises 10 to 100 % by weight, preferably 30 to 90 % by weight and more preferably 50 to 80% by weight of solubilising lipid.

Further to the amphiphilic lipid and to the solubilising lipid, the oily phase prepared in step A1a) (method A) or step B1) (method B) can comprise other components, such as an oil, an imaging agent, a therapeutic agent, a cationic surfactant, an anionic surfactant or a mixture thereof, as described above.

In general and when present, the oil is contained in the oily phase in a proportion ranging from 5 to 80 % by weight, preferably between 10 and 50 % by weight and more preferably 10 to 30 % by weight.

When present, the amount of therapeutic agent is dependent on the targeted application and the type of agent. However it is generally sought to formulate the nanoparticles with a maximum concentration of therapeutic agent, in particular if they are scarcely soluble therapeutic agents to limit the volume and/or period of administration to the patient. Generally, the oily phase comprises a quantity of 0.001 to 10 % by weight, preferably 0.01 to 5 % by weight, or even more preferably 0.1 to 3 % by weight of therapeutic agent.

Yet it has been found that the presence of the solubilising lipid in the nanoparticles allows the incorporation of a high quantity of therapeutic agents, even hydrophobic or amphiphilic therapeutic agents.

Typically, when the oily phase comprises a cationic surfactant, the process according to the invention comprises, after step A1c), step A1d), or step A2) (preferably after step A1c) and before step A2)) (method A), or after step B3) (method B), a step C) of contacting the material in hydrogel form with nucleotide sequences, such as DNA, mRNA or a nucleotide sequence able to modulate endogenous mechanisms of RNA interference, preferably as defined above, under conditions that allow for the loading of said nucleotide sequence onto the nanoparticles. Step C) is performed before step 3) of drying. This allows obtaining a material comprising a polymer capable of forming a hydrogel and nanoparticles the surface of which has been loaded with a nucleotide sequence able to modulate endogenous mechanisms of RNA interference. The nucleotide sequences are maintained on the surface of the nanoparticles by means of electrostatic interactions with the cationic surfactant. They are therefore located on the surface of the nanoparticles. The nucleotide sequences are loaded to the nanoparticles following the procedure described in application WO 2014/032953 (see pages 41 to 44 thereof).

Mixing can optionally be facilitated by placing one of the constituents or the mixture in solution in a suitable organic solvent. The organic solvent is then evaporated to obtain a homogenous oily phase.

Also, it is preferable to conduct the mixing at a temperature at which all the constituents are liquid.

- Step A1 b) (method A) or step B2) (method B): Preparing an aqueous phase

The aqueous phase is preferably water and/or a buffer such as a phosphate buffer e.g. PBS ("Phosphate Buffer Saline"), acetate buffer, or a saline solution in particular sodium chloride.

The proportion of oily phase and aqueous phase is most variable. However, most often, 1 to 50 %, preferably 5 to 40 % and more particularly 10 to 35 % by weight of oily phase compared to the total weight of the oily phase and of the aqueous phase (without taking into account the polymer capable of forming a hydrogel weight in the aqueous phase weight as regards step B2)).

The aqueous phase comprises a co-surfactant and optionally a functionalizable surfactant.

In step A1b) (method A), the aqueous phase is generally free from polymer capable of forming a hydrogel. On the contrary, in step B2) (method B), the aqueous phase comprises a polymer capable of forming a hydrogel.

Although the co-surfactant is introduced in the aqueous phase, the co-surfactant is located in the nanoparticles obtained at the end of steps A1c) or step B3). In the present application, the weight of nanoparticles thus includes the weight of co-surfactant.

The aqueous phase generally comprises 0.01 to 50 % by weight, preferably 0.5 to 30 % by weight, and in particular 1 to 25 % by weight of a co-surfactant.

Typically, when the aqueous phase comprises a functionalizable surfactant, the process according to the invention comprises after step A1c) or step A2) (method A) or after step B3) (method B) a step C') of contacting the material in hydrogel form with a therapeutic agent, preferably as defined above, under conditions that allow for the reaction of the functionalizable surfactant and the therapeutic agent. Step C') is performed before step 3) of drying. This allows obtaining a material comprising a polymer capable of forming a hydrogel and nanoparticles the surface of which has been grafted with a therapeutic agent. Generally, the therapeutic agent is covalently bonded to the surface of the nanoparticles.

This embodiment of the process is an alternative to the embodiment described above wherein the co-surfactant of the aqueous phase used in the process comprises a therapeutic agent. Both embodiments allow obtaining a material comprising a polymer capable of forming a hydrogel and nanoparticles the surface of which has been grafted with a therapeutic agent. The only difference is when the therapeutic agent is introduced in the nanoparticles, either by the formation of the nanoparticles are formed (in the embodiment wherein the co-surfactant comprises a therapeutic agent),

or by a subsequent step C) defined above allowing the grafting of the therapeutic agent to previously formed nanoparticles the surface of which comprise functionalizable groups thickening agent

The use of an aqueous phase of higher viscosity facilitates emulsification and thereby allows a reduction in sonication time.

The aqueous phase advantageously comprises 0 to 75 % by weight, preferably 0 to 60 % by weight of thickening agent.

The aqueous phase may further contain other additives such as dyes, stabilisers, pH adjusting agent and/or preserving agents. For example, the aqueous phase comprises acetic acid which is useful as preservative and as pH adjuster. Preferably, the pH of the aqueous phase of step A1) or of step B2) is from 2.5 to 9.0, more preferably from 3.0 to 8.5, for example from 3.5 to 7.5.

The aqueous phase prepared in step B2) of method B comprises a polymer capable of forming a hydrogel.

The polymer capable of forming a hydrogel is preferably in hydrogel form when it is introduced into the aqueous phase in step B2). Typically, this hydrogel comprises (or is constituted from) the polymer capable of forming a hydrogel and an aqueous solution, such as a buffer, for example an acetate buffer. Preferably, this hydrogel comprises from 0,1 to 20% by weight of the polymer capable of forming a hydrogel, more preferably from 0.5 to 10.0% by weight, for example 1% to 7.5% by weight. In another embodiment, the polymer capable of forming a hydrogel is in solid form when it is introduced into the aqueous phase in step B2).

Generally, the weight of polymer capable of forming a hydrogel used in step B2) is calculated so that the weight of polymer capable of forming a hydrogel compared to the total weight of the nanoparticles components (wherein the nanoparticles components comprise the amphiphilic lipid, the solubilising lipid, the co-surfactant, the optional oil, the optional imaging agent, the optional therapeutic agent, the optional cationic surfactant, the optional anionic surfactant, the optional helper lipid) is from 0.05/1 to 1/1, preferably from 0.05/1 to 0.2/1. Of course, when the polymer capable of forming a hydrogel used in step B2) is in hydrogel form, the weight of polymer capable of forming a hydrogel above corresponds to dry polymer capable of forming a hydrogel.

• Step A1c) (method A) or step B3) (method B): Dispersing the oily phase in the aqueous phase

Step A1c) (method A) or step B3) (method B) consists in dispersing the oily phase in the aqueous phase under the action of sufficient shear to form nanoparticles.

Advantageously the oily phase is dispersed in the aqueous phase in the liquid state. If one of the phases solidifies at room temperature (20°C), it is preferable to perform mixing by heating one or preferably both phases to melting temperature or higher.

Forming the nanoparticles under shear effect is preferably performed using a sonicator, a microfluidiser or a high pressure homogenizer.

Forming the nanoparticles under shear effect in step A1c) (method A) is preferably performed using a sonicator or a high pressure homogenizer. For sonication, preferably the aqueous phase and then the oily phase are added in the desired proportions to a cylindrical container and the sonicator is immersed in the centre thereof and set in operation for sufficient time to obtain nanoparticles, most often for a few minutes. For High Pressure Homogenization, typically, the aqueous phase and the oily phase are crudely mixed in order to pre-form a coarse emulsion, which is then used to fill the tank of the homogeneizer. The emulsion is then processed with the homogeneizer using the number of cycles necessary to achieve the targeted size for the nanoparticles.

Forming the nanoparticles under shear effect in step B3) (method B) is preferably performed using a high pressure homogenizer. Indeed, a degradation of the polymer might be observed when a sonicator is used instead.

At the end of step A1c) (method A) or step B3) (method B), the diameter of the obtained nanoparticles is generally greater than 10 nm and smaller than 500 nm, preferably between 20 and 400 nm, most preferably between 30 and 300 nm.

Advantageously, at the end of step A1c) (method A), when the nanoemulsion has been prepared from an oily phase free of cationic or anionic surfactant, a nanoemulsion is obtained in which the absolute value of the zeta potential of the surface of the nanoparticles is generally lower than 20 mV, preferably when the aqueous phase is PBS 0.1x.

For example, the mean diameter and/or zeta potential can be determined by dynamic light scattering (DLS), for example with a Zeta Sizer Nano ZS Malvern Instrument.

• Step A2) (method A): mixing the nanoemulsion obtained in step A1) with a polymer capable of forming a hydrogel to obtain the material in hydrogel form.

The polymer capable of forming a hydrogel used in step A2) is generally chosen from the polymers capable of forming a hydrogel described above for step B2 of method B.

The polymer capable of forming a hydrogel is preferably in hydrogel form when it is introduced into the nanoemulsion in step A2). Typically, this hydrogel comprises (or is constituted from) the polymer capable of forming a hydrogel and an aqueous solution, such as a buffer, for example an acetate buffer. Preferably, this hydrogel comprises from 0,1 to 20% by weight of the polymer capable of forming a hydrogel, more preferably from 0.5 to 10% by weight, for example 1% to 7.5% by weight. In another embodiment, the polymer capable of forming a hydrogel is in solid form when it is introduced into the nanoemulsion in step A2).

Generally, the weight of polymer capable of forming a hydrogel used in step A2) is calculated so that the weight of polymer capable of forming a hydrogel compared to the total weight of the nanoparticles components (wherein the nanoparticles components comprise the amphiphilic lipid, the solubilising lipid, the co-surfactant, the optional oil, the optional imaging agent, the optional therapeutic agent, the optional cationic surfactant, the optional anionic surfactant, the optional helper lipid) is from 0.5/1 to 1000/1, preferably from 1/1 to 500/1, most preferably from 2/1 to 500/1. Of course, when the polymer capable of forming a hydrogel used in step B2) is preferably in hydrogel form, the weight of polymer capable of forming a hydrogel above corresponds to dry polymer capable of forming a hydrogel.

• Optional subsequent steps after step A1d) (method A)

The nanoemulsion obtained at the end of step A1d) (method A) can be purified, for example by column purification or dialysis.

The nanoemulsion obtained at the end of step A1d) (method A) can be diluted and/or sterilised, for example by filtration or dialysis. This step allows the removal of any aggregates which may have been formed during preparation of the nanoparticles.

• Optional additional steps

• step 2): Applying the material in hydrogel form obtained at the end of step A2) or B3) to a substrate

In one embodiment, the process comprises a step 2) of applying the material in hydrogel form obtained at the end of step A2) or B3) to a substrate. For example, glass substrates or polymeric substrates such as polyurethane foam substrates can be used.

The application can be carried out by spraying, coating, rolling, dip-coating...

The material in hydrogel form can be applied to the totality or a part of the surface of the substrate. It is thus possible to apply the material in hydrogel form to a specific pattern, the form of which being adjustable depending on the intended use of the material.

• Step 3): Drying the material in hydrogel form obtained at the end of step A2) or B3)

In one embodiment, the process comprises a step 3) consisting in drying the material in hydrogel form obtained at the end of step A2) or B3), preferably to obtain the material comprising less than 25%wt of water. Drying can be carried out by freeze-drying or by evaporating the water in an oven, for example at temperatures between 30°C to 90°C, preferably between 40°C and 60°C, for example 50°C. Preferably, drying is carried out by freeze drying (also called lyophilisation).

In the embodiment wherein the process comprises step 2), the material obtained at the end of step 3) is supported on the substrate.

A fourth object of the invention is a material comprising a polymer capable of forming a hydrogel and nanoparticles obtainable (or obtained) by the process described above.

**[Use of the material]**

A fifth object of the invention is the use of the material comprising a polymer capable of forming a hydrogel and nanoparticles described above for wound healing dressings, cell culture materials, tissue engineering, implants, patches, coatings (for example coatings of solid surface, such as needle or microneedle), sensors, in diagnostic, pharmaceutical, or cosmetic industry.

A sixth object of the invention is a wound healing dressing, a 3D cell culture material, or a tissue comprising the material comprising a polymer capable of forming a hydrogel and nanoparticles described above.

As regards the use of the material as a wound healing dressing, the nanoparticles loaded on the polymer capable of forming a hydrogel of the material advantageously possess by themselves beneficial effects for skin and tissue repair.

Advantageously, the material is not cytotoxic.

• Material for the delivery of the therapeutic

When the nanoparticles of the material comprise a therapeutic agent, the material is also useful for the delivery of the therapeutic agent, in particular for the prevention and/or treatment of a disease.

Despite the huge potential of hydrogels described in the literature, in many cases these systems have limited use as for drug delivery, since they exhibit poor mechanical properties, relatively rapid release of drugs from the hydrogel (particularly in the case of hydrophilic drugs), and/or inefficient hydrophobic drug loading. Nanocomposite hydrogels, i.e., molecular networks physically or covalently cross-linked with nanoparticles, have been proposed as innovative means to overcome some of the challenges associated with the use of hydrogels in drug delivery systems, as disclosed in Merino et al. (ACSNano, 9(5), 4686-4697, 2015).

The material the nanoparticles of which comprise a therapeutic agent is useful as drug delivery system, being biocompatible and allowing the improvement of the bioavailability of the drug. It is possible to control the release of the therapeutic agent by tailoring the concentration and components of the material.

Preferably, the material is used for the delivery of the therapeutic agent via topical or subcutaneous route.

A method for the prevention and/or treatment of a disease comprising the administration to an animal, generally a mammal, preferably a human in need thereof, of an efficient quantity of the material comprising a polymer capable of forming a hydrogel and nanoparticles comprising a therapeutic agent such as defined above is also one of the subjects of the present invention.

Encapsulating or loading the therapeutic agent into/onto the nanoparticles of the material is beneficial in comparison to direct loading of the therapeutic agent in the matrix of the polymer capable of forming hydrogel for the following reasons:

- it helps solubilizing poorly water-soluble therapeutic agents,
- it allows controlling the kinetics of release of the therapeutic agent by controlling the kinetics of release of the nanoparticles through their interactions with the matrix of the polymer capable of forming hydrogel
- it benefits of the vectorization properties of the nanoparticles.

The material acts as a reservoir of therapeutic agents which are released as the material degrades. Thus, the bioavailability of the therapeutic agent is improved over time.

In one embodiment, the material comprises a therapeutic agent in the nanoparticles and a therapeutic agent of different nature in the polymer matrix.

This allows the delivery of therapeutic agents with different kinetics of release. The therapeutic agent in the polymer matrix is indeed delivered more rapidly than the therapeutic agent in the nanoparticles.

For example, the material comprises a therapeutic agent in the nanoparticles and an anaesthetic or an analgesic agent in the polymer matrix.

• Material for as material for cell culture applications

The material the nanoparticles of which comprise a therapeutic agent chosen from hormones, vitamins, saccharides, growth factors, cell adhesion factors, cell migration factors, chemotherapeutic agents, antibiotics, antimicrobial agents, nucleotide sequences, such as DNA or nucleotide sequences able to modulate endogenous mechanisms of RNA interference for example siRNA, is useful as material for cell culture, in particular for cell culture in gel matrix.

In an embodiment, the material comprises different types of nanoparticles, each type of nanoparticle comprising a growth factor of different nature. This embodiment is advantageous for the progressive release of the growth factors depending on the cell growth stage, the nanoparticles being delivered from the degradation of polymers composing the material, and then being taken up by cells for better acting on their receptors and the associated signalling pathways. Several growth factors can be incorporated into such materials containing nanoparticles. The embodiment allows thus a better protection of the growth factors in the reservoir from extracellular degradation, rendering them available on demand.

• Material for dressing, implant or implant coating applications

When the nanoparticles of the material comprise a therapeutic agent chosen from chemical compounds capable of promoting wound healing, growth factors, antibiotics, antimicrobial agents, saccharides, and nucleotide sequences, such as DNA, mRNA or nucleotide sequences able to modulate endogenous mechanisms of RNA interference for example SiRNA, the material is also useful as dressing, as implant or as implant coating (for example useful to induce tissue reconstruction).

• Material for vaccine application

In one embodiment, the material comprises nanoparticles comprising one or several antigens or a nucleic acid molecule encoding one or several protein antigens as therapeutic agent(s). This material can be used in the methods described hereafter. This material can be either a vaccine patch for topical or sublingual delivery of the antigen(s) or nucleic acid molecule, or coated onto a needle or a microneddle array for injection or transdermal delivery of the nanoparticle content, or a subcutaneous implant for a sustained delivery of the antigen(s) or nucleic acid molecule.

The invention concerns a method for eliciting improved antibodies-mediated and/or cell-mediated immune responses after immunization protocol of animals or humans by the use of this material, and possibly a better protection against the considered disease.

The invention concerns also a method for the bioproduction of polyclonal or monoclonal antibodies in animals or cells by use of the material.

Thus, the invention concerns a method for the production of polyclonal antibodies, comprising the steps consisting in:

(a) immunization of an animal with the material, whereby a humoral immune response against the antigen is induced, and

(b) recovering the polyclonal antibodies directed against the antigen.

The invention also concerns a method for the production of monoclonal antibodies, comprising the steps consisting in:

(i) immunization of an animal with the material,

(ii) recovering and isolating the B lymphocytes from the animal immunized at step (i),

(iii) producing an hybridoma and culturing the hybridoma, in order to produce monoclonal antibodies directed against the antigen,

(iv) recovering and purifying the monoclonal antibodies produced at step (iii).

The immunization at steps (a) and (i) is carried out by injecting the material to an animal at a dosage effective to induce a humoral immune response to the antigen.

The animal is usually a rodent (mouse, rat, hamster), a rabbit, a chicken, a goat, a sheet, a monkey, a horse, a cavia porcellus or a hen.

In the method for the production of polyclonal antibodies, step (b) of recovering the polyclonal antibodies directed against the antigen is carried out by usual techniques, comprising collecting the blood of the immunized animal (with or without killing), followed by isolating the serum containing polyclonal antibodies, and optionally by purifying the polyclonal antibodies.

In the method for the production of monoclonal antibodies, recovering and isolating the B lymphocytes at step (ii) is carried out by usual techniques, notably killing the immunized animal, followed by taking out the spleen and isolating the B lymphocytes from the spleen. Producing a hybridoma and culturing the hybridoma at step (iii) is carried out by usual techniques, and comprises the fusion of the B lymphocytes isolated at step (ii) with a myeloma, in order to produce a hybridoma. Fusion is for example carried out by use of polyethylene glycol or by electroporation. The thus obtained hybridoma is then cultivated in appropriate conditions, that can be easily determined by the skilled person in order to allow the hybridoma to produce antibodies. In last step (iv), the monoclonal antibodies are recovered and purified, for example by high-performance liquid chromatography, by affinity chromatography based on G protein, or by precipitating with ammonium.

The invention also concerns the material for its use to induce humoral response and/or cell-mediated immune responses against one or several antigens present in the material or against the antigen encoded by the nucleic acid molecule present in the material, preferably in an animal to which the material is administered.

The invention also concerns a method of immunization against a disease, comprising administering the material to an animal, preferably a human, in need thereof.

Preferably, the disease is an infection, an allergy, an inflammatory disease, an auto-immune disease or a cancer. Preferably, the infection is a fungal, bacterial, viral, or parasitic infection caused by a pathogen from which the antigens are derived. Preferably, the inflammatory disease is chosen from atherosclerosis, myocardial ischemia, acne, asthma, auto-immune diseases, prostatitis, la glomerulonephritis, hypersensibilities, chronic intestinal inflammatory diseases, pelvic inflammatory diseases, rheumatoid polyarthritis, graft rejection, vasculitis, interstitial cystitis, allergies and inflammatory myopathies. Preferably, when the disease is cancer, the antigen is a tumor associated antigen (TAA), and the cancer is a cancer from which these tumor antigens are derived.

The invention also concerns the material comprising nanoparticles comprising one or several antigens and/or

molecules acting on the immune system (adjuvant molecules, TLR agonists...) as therapeutic agents for its use as vaccine.

• Material useful as transfecting agent

When the nanoparticles of the material comprise a nucleotide sequence able to modulate endogenous mechanisms of RNA interference, the material is also useful:

- as transfecting agent, i.e. for inserting nucleotide sequences able to modulate endogenous mechanisms of RNA interference into a eukaryote cell, and/or
- for the *in vitro* or *in vivo* delivery of nucleotide sequences able to modulate endogenous mechanisms of RNA interference, in particular for the prevention and/or treatment of a disease for which the silencing of one or more genes is sought.

For example, the material the nanoparticles of which comprise a nucleotide sequence able to modulate endogenous mechanisms of RNA interference can be introduced, preferably in a dry form, in the wells of a microassay plate useful for transfection. The user just has to introduce the cells to be transfected into the wells and incubate them in the appropriate experimental conditions for having an efficient siRNA transfection.

The invention is also directed to a microassay plate comprising wells, wherein each well comprises the material the nanoparticles of which comprise a nucleotide sequence able to modulate endogenous mechanisms of RNA interference, the material being preferably in dry form.

In one embodiment, the nature of the nucleotide sequences able to modulate endogenous mechanisms of RNA interference of the nanoparticles of the material are different in each line of wells of the plate, or in each well of the plate. The plate is useful for transfecting.

In another embodiment, the nature of the nucleotide sequences able to modulate endogenous mechanisms of RNA interference of the nanoparticles of the material are identical in each well of the plate. The plate is useful for transfecting or for downregulating a specific protein, for example EGF protein. Various cell types can be tested.

**[Definitions]**

In the present application, by the term « nanoemulsion » is meant a composition having at least two phases, an oily phase and an aqueous phase in which the mean size of the nanoparticles of the dispersed phase is smaller than 1 micron, preferably 10 to 500 nm and in particular 20 to 400 nm, and most preferably 30 to 300 nm. The nanoemulsion is an oil-in-water emulsion. It can be simple or double (i.e. the dispersed phase may comprise an internal aqueous phase). It is preferably simple.

In the meaning of the present application, the expression « dispersed phase » designates the nanoparticles comprising the solubilising lipid / the amphiphilic lipid / the co-surfactant / the optional oil / the optional imaging agent / the optional therapeutic agent the optional cationic surfactant / the optional helper lipid. The dispersed phase is generally free of aqueous phase.

The term « nanoparticle encompasses both the nanoparticles (also called droplets) of liquid oil and the solid nanoparticles derived from emulsions of oil-in-water type in which the dispersed phase is solid. In the present application, the term "nanoparticle" is used both for the nanoparticles of the dispersed phase of the nanoemulsion/ material in hydrogel form and for the nanoparticles after the drying step (i.e. in the absence of aqueous phase).

The term « lipid » in this description designates all fatty bodies or substances containing fatty acids present in the fats of animal origin and in vegetable oils. They are hydrophobic or amphiphilic molecules chiefly formed of carbon, hydrogen and oxygen and having a density lower than that of water. The lipids may be in the solid state at room temperature (25°C) as in waxes, or liquid state as in oils.

The term « phospholipid » concerns lipids having a phosphate group, in particular phosphoglycerides. Most often the phospholipids comprise a hydrophilic end formed by the optionally substituted phosphate group and two hydrophobic ends formed by fatty acid chains. Amongst the phospholipids, particular mention is made of phosphatidylcholine, phosphatidyl ethanolamine, phophatidyl, inositol, phosphatidyl serine and sphingomyeline.

The term « lecithin » designates phosphatidylcholine i.e. a lipid formed from a choline, a phosphate, a glycerol and from two fatty acids. It more broadly covers living phospholipid extracts of vegetable or animal origin, provided that they are mostly formed of phosphatidylcholine. These lecithins generally form mixtures of lecithins carrying different fatty acids.

The term « fatty acid » designates aliphatic carboxylic acids having a carbon chain of at least 4 carbon atoms. Natural fatty acids have a carbon chain of 4 to 28 carbon atoms (generally an even number). The term long chain fatty acid is used for a length of 14 to 22 carbons and very long chain if there are more than 22 carbons.

By the term « surfactant » is meant compounds with amphiphilic structure imparting particular affinity thereto

for interfaces of oil/water and water/oil type providing them with the capability of reducing the free energy of these interfaces and stabilising dispersed systems.

By the term « co-surfactant » is meant surfactant acting in addition to a surfactant to further reduce interface energy.

By the term "hydrogel" is meant a three-dimensional water-swollen cross-linked polymeric structure containing (1) covalent bonds produced by the reaction of one or more comonomers, (2) physical cross-links due to chain entanglements, (3) association bonds including hydrogen bonds or strong van der Waals interactions between chains, and/or (4) crystallites bringing together two or more macromolecular chains. Although the polymer chains (or monomers) constituting the hydrogel are usually hydrophilic and often water soluble, a hydrogel is insoluble in water due to the presence of these chemical or physical crosslinks between the polymer chains or monomers). A hydrogel is capable of imbibing large amounts of water, generally capable of retaining at least 1 time, typically 2 times, preferably 5 times, for example 10 times its dried weight of water.

By the expression "polymer capable of forming a hydrogel" is meant a hydrophilic polymer, which, after chemical or physical crosslinking, can swell in water as a result of absorption of water by the polymer network. The polymer in the form of a hydrogel (swollen state) comprises more than 50% wt of aqueous phase.

By the expression "nanoparticles homogeneously distributed within the polymer" is meant that the nanoparticles are distributed evenly and uniformly within the entire material. The concentration of the nanoparticles is substantially the same within the entirety of the material. As used herein, a concentration is "substantially the same" if the concentration does not fluctuate by more than +/-25%, preferably +/-10%, throughout the entirety of the volume in which the concentration is measured. For example, the concentration of nanoparticles can be investigated using Scanning Electron Microscopy (SEM) images or Transmission Electron Microscopy (TEM) images to calculate at length scales on the order of 4 $mm^2$ the number of nanoparticles inside the material. It can also be possible to quantify the number of nanoparticles released from pieces of materials on the order of 10 $mm^3$ using chromatography quantitation.

The invention is descried in better detail with reference to the appended Figures and following examples.

## FIGURES

- Figure 1: Photograph of the chitosan film encapsulating nanoparticles prepared in example 1.2..
- Figures 2-13: SEM photographs of the different materials loaded with curcumin of example 4.
  In Figures 2, 6 and 10, the material is the collagen comprising curcumin material (free from nanoparticles) (control) with collagen/curcumin 10/0.01 weight/weight.
  In Figures 3, 7 and 11, the material is the collagen/F50 nanoparticles comprising curcumin 10/1/0.01 collagen/lipids/curcumin w/w/w material.
  In Figures 4, 8 and 12, the material is the collagen/F80 nanoparticles comprising curcumin 10/1/0.01 collagen/lipids/curcumin w/w/w material.
  In Figures 5, 9 and 13, the material is the collagen/F120 nanoparticles comprising curcumin 10/1/0.01 collagen/lipids/curcumin w/w/w) material.
  In Figures 2 to 5, SEM photographs of the cross sections of the different materials are represented.
  In Figures 6 to 9, SEM photographs of the lower surfaces of the cross sections of the different materials are represented.
  In Figures 10 to 13, SEM photographs of the upper surfaces of the cross sections of the different materials are represented.
  For figures 2-13, the magnification is 650, the scale bar represents 20 μm.
- Figures 14-21: TEM images of the different materials loaded with curcumin of example 4.
  In Figures 14 and 18, the material is the collagen comprising curcumin material (free from nanoparticles) (control) with collagen/curcumin 10/0.01 weight/weight. In Figure 14, scale bar represents 5 μm. In Figure 18, scale bar represents 1 μm.
  In Figures 15 and 19, the material is the collagen/F50 nanoparticles comprising curcumin 10/1/0.01 collagen/lipids/curcumin w/w/w material. In Figure 15, scale bar represents 2μm. In Figure 19, scale bar represents 500 nm.
  In Figures 16 and 20, the material is the collagen/F80 nanoparticles comprising curcumin 10/1/0.01 collagen/lipids/curcumin w/w/w material. In Figure 16, scale bar represents 2 μm. In Figure 20, scale bar represents 500 nm.
  In Figures 17 and 21, the material is the collagen/F120 nanoparticles comprising curcumin 10/1/0.01 collagen/lipids/curcumin w/w/w material. In Figure 17, scale bar represents 2 μm. In Figure 21, scale bar represents 500 nm.
- Figure 22. Gel assays to assess nanoparticle/SiRNA complexation (example 6). Figure 21 A: for F50/SiRNA complexes; Figure 21 B: for F130/SiRNA nanoparticles.

## EXEMPLES

### Example 1: nanoparticles loaded in chitosan films

1.1 Preparation of F50 nanoemulsions

Oil-in-water emulsions comprising nanoparticles having 50 nm diameters and comprising the compounds listed in Table 1 were prepared.

Table 1: Composition of the F50 emulsions.

| Compound | Provider | Quantity (mg) | | |
|---|---|---|---|---|
| | | "Neutral" F50 emulsion | "Cationic" F50 emulsion | "Anionic" F50 emulsion |
| refined soybean oil | Croda Uniqema, France | 85 | 67.2 | 85 |
| Suppocire NC™ | Gatefosse, France | 255 | 22.4 | 255 |
| Dil dye as therapeutic agent model | Life Technologies | 0.75 | 0.75 | 0.75 |
| Lipoid S75™ | Lipoid, Germany | 65 | 2.2 | 33 |
| DOTAP (cationic surfactant) | Avanti Polar, USA | - | 15 | - |
| Hostaphat CC100 (anionic surfactant) | Clariant | - | - | 32 |
| Myrjs40™ (neutral surfactant) | Croda Uniqema, France | 345 | 43.1 | 345 |
| Glycérol | Sigma Aldrich | - | 1000 | - |
| aqueous medium: 154 mM NaCl | | qsp 2 ml | qsp 2 ml | qsp 2 ml |

An oily premix was prepared by mixing refined soybean oil, Suppocire NC™, Lipoid S75™, DOTAP, Hostaphat CC100 and Dil dispersed in ethanol as therapeutic agent model. After homogeneization at 50°C and complete evaporation of ethanol, the continuous aqueous phase, composed of Myrjs40™ and of aqueous medium, eventually added with glycerol, was introduced. The vial was placed in a 50°C water bath and the mixture was sonicated for 5 min using a VCX750 ultrasonic processor (power output 190 W, 3-mm probe diameter, Sonics). The obtained emulsions were dialyzed overnight at room temperature (about 20°C) (20°C) against 1000 times their volume in the appropriate aqueous buffer (12-14,000 Da MW cut off membranes, ZelluTrans).

Finally, the concentration of the emulsion was adjusted to 100 mg/mL (10% w/w) of lipids in 1X PBS (lipids include oil + Suppocire + Lipoid + Myrjs40 + DOTAP + Hostaphat CC 100), and filtered through a 0.22 μm Millipore membrane for sterilization.

The emulsions were characterized by their size and polydispersity index, using Dynamic Light Scattering (Zeta Sizer Nano ZS, Malvern Instrument). For this purpose, the emulsions were diluted to achieve 2 mg/ml of lipids in sterile PBS 0.1X and were transferred in Zeta Sizer Nano cells (Malvern Instrument) before each measurement, performed in triplicate. Zeta potential measurements were performed in NaCl 0.154 mM.

Results are described in Table 2.

Table 2. Nanoparticle diameter, PdI and zeta potential of the prepared emulsions.

| Emulsion | Nanoparticles concentration (mg/mL) | Nanoparticle diameter (nm) | Polydispersity Index (PdI) | Zeta potential (mV) |
|---|---|---|---|---|
| "Anionic" F50 | 100 | 58.4 | 0.148 | - 12.4 |

(continued)

| Emulsion | Nanoparticles concentration (mg/mL) | Nanoparticle diameter (nm) | Polydispersity Index (PdI) | Zeta potential (mV) |
|---|---|---|---|---|
| "Neutral" F50 | 100 | 52.3 | 0.117 | - 5.7 |
| "Cationic" F50 | 100 | 65.7 | 0.144 | + 14.3 |

1.2. Preparation of chitosan films including F50 nanoemulsions

Chitosan films were prepared using either a low molecular weight chitosan (noted LWM-CS, Sigma-Aldrich reference 740063, 86 kDa), or a high molecular weight chitosan (noted HMW-CS, Protosan, reference B80/500 FA 412-08, 300 kDa). 5 mL of a chitosan solution at 10 mg/mL in an aqueous 1% v/v acetic acid solution (pH 4) were added with 5 mg of nanoparticles (50 μl of a 10% w/w PBS solution) and poured in PDMS molds of 5 cm long x 2 cm large x 2 cm high. The solution was then dried at 50°C for 4h. The films were then immersed for 3 minutes in ammonium hydroxide 1.2% v/v and rinsed in deionized water. Regular translucent films including 10% w/w of F50 nanoparticles were therefore obtained as shown in Figure 1. Films of pure chitosan material (ie non including nanoparticles) were prepared as well using the same process.

The dried films were characterized for their thickness (Table 3).

Table 3. Thickness of the dried chitosan films.

| Material | Thickness (μm) |
|---|---|
| LWM-CS | 36 ± 16 |
| LWM-CS / F50 "neutral" nanoparticles | 38 ± 6 |
| HWM-CS | 37 ± 6 |
| HWM-CS / F50 "neutral" nanoparticles | 41 ± 5 |

The loading of the films by the nanoparticles did not significantly modify the width of the films, even for high payloads in nanoparticles (50% w/w nanoparticle/polymer).

Swelling ratios of the chitosan films were measured. For these purpose, pieces of films (about 1 cm x 1 cm) of approximately 10 mg dried weight (which were accurately measured to obtain the polymer weight) were poured for 4 h in 2 mL of 1X PBS. In buffer medium, they swell to form a thin hydrogel layer encapsulating the aqueous medium. At the end of the swelling, the material was put on a filter paper for 1 min to absorb excess of buffer then weight to obtain the swollen weight of the material. The swelling ratios measured as

$$SR = 100 \times (W_w - W_d) / W_d$$

in which $W_d$ is the weight of material (dry material) and $W_w$ is the material after 4 hours immersion in 1X PBS are reported in Table 4.

Table 4. Swelling ratios of the low molecular weight chitosan films.

| Material | Swelling ratio |
|---|---|
| LWM-CS | 130 ± 20 |
| LWM-CS / F50 "neutral" nanoparticles | 240 ± 30 |
| LWM-CS / F50 "anionic" nanoparticles | 140 ± 20 |
| LWM-CS / F50 "cationic" nanoparticles | 240 ± 60 |

The swelling ratio of the films was the same with and without anionic nanoparticles. Loading the films with

neutral or cationic nanoparticles however increased their swelling ratio by 85 %.

1.3. Release of nanoparticles from (chitosan films including F50 nanoemulsions)

Nanoparticle release from the films when immersed in 1X PBS was estimated based on the fluorescence quantification of the Dil dye encapsulated in the nanoparticles, entrapped in the chitosan films. Film pieces (1 cm x 1 cm) were immersed in 2 mL PBS 1X and incubated at room temperature (about 20°C) in dark. At desired time points, films were dried on a filter paper for a few seconds then observed by fluorescence microscopy. Films were observed with a Axio Vert A1 Zeiss microscope at 20 x magnification with 558 nm excitation and exposure time of 200 ms. The % of released nanoparticles from the film was estimated as the % of fluorescence loss of the materials.

Results are reported in Table 5.

Table 5. % of nanoparticles released when incubating pieces of films (1cm x 1cm) in 2 mL of 1X PBS at room temperature (about 20°C).

| Time (days) | % of "neutral" nanoparticles released from the chitosan film | % of "anionic" nanoparticles released from the chitosan film | % of "cationic" nanoparticles released from the chitosan film |
|---|---|---|---|
| 0 | 0 | 0 | 0 |
| 1 | 19.0 ± 2.4 | 11.2 ± 0.3 | 33.2 ± 2.1 |
| 6 | 22.8 ± 1.1 | 47.7 ± 6.9 | 11.5 ± 1.0 |
| 8 | 39.8 ± 16.6 | 74.6 ± 0.6 | 27.1 ± 2.6 |
| 9 | 64.3 ± 5.9 | 80.7 ± 0.9 | 20.1 ± 2.9 |
| 10 | 57.9 ± 5.6 | 63.0 ± 3.2 | 28.8 ± 1.2 |

1X PBS buffer has a pH around 7.4 and chitosan is neutral around this pH (pKa of chitosan is around 6.6-6.8 at room temperature). The kinetics of release from the neutral matrix was not governed by the nanoparticle charges as "neutral" and "anionic" nanoparticles, of similar hydrodynamic diameter (about 55 nm), displayed similar kinetics of release. We observed that cationic nanoparticles, with a larger diameter (66 nm), were released more slowly from the material.

**Example 2: nanoparticles loaded in chitosan-PEG hydrogels**

2.1. Preparation of F50 nanoemulsions

Same nanoparticles as example 1.1.

2.2. Preparation of chitosan-PEG hydrogels including F50 nanoemulsions

Chitosan-PEG hydrogels including or not F50 nanoparticles (either neutral, anionic or cationic) at a 10% w/w or a 50% w/w nanoparticle/chitosan payload were prepared by cross-linking under UV light in the presence of a photoinitiator (Irgacure 2959), chitosan modified by alkene groups and a SH-PEG-SH polymer, according to Scheme I.

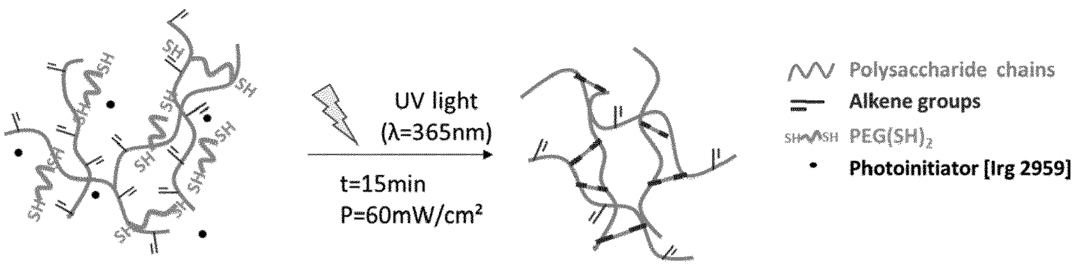


Scheme I. Cross-linking reaction activated by UV light between polysaccharide bearing alkene groups ad SH-PEG-SH (thiol-ene coupling reaction).

2.2.1. Synthesis of chitosan-pentenoate

Alkene groups were introduced on chitosan (Sigma-Aldrich reference 740063, 86 kDa) by reacting 4-pentenoïc anhydride with the polysaccharide, according to Scheme II.

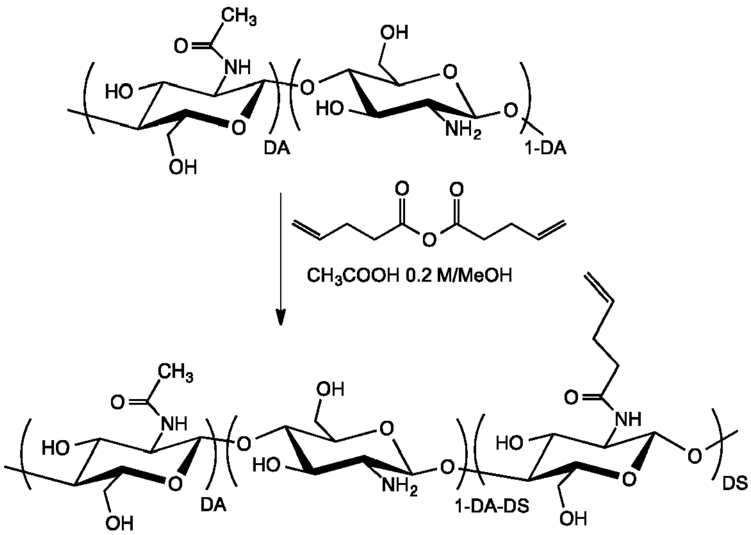


Scheme II. Chemical modification of chitosan. DA: degree of acetylation; DS: degree of substitution.

First, chitosan (0.4 g) was dissolved in 90 mL of 0.2 M acetic acid and stirred at 4°C overnight. 90 mL of methanol were added ([CS] = 2.2 g/L) and the solution stirred for 1 hour. 88 mg (0.2 eq.) of 4-pentenoic anhydride (Sigma-Aldrich) were diluted in 1 mL methanol then added dropwise to the chitosan solution and reacted for 2 hours. After reaction, modified chitosan was precipitated by addition of NaOH (1 M), then washed by ethanol/water mixtures with increasing ethanol gradient (3/2 EtOH/$H_2O$, 7/3 EtOH/$H_2O$, 9/1 EtOH/$H_2O$, EtOH). At the end, the lumpy material was lyophilized (Labconbo freezone 6 lyophilisator, P = 0.05 mBar, T = -50°C) to obtain a white powder.

$^1H$ NMR spectra of the chitosan-pentenoate dissolved in deuterium oxide (6 mg/mL) were performed at 80 °C using a Bruker DRX400 spectrometer operating at 400 MHz. All spectra were recorded by applying a 45° tip angle for the excitation pulse, and a 10 s recycle delay for 16 scans. The degree of substitution (DS) of the samples was determined by digital integration of the anomeric protons signals of the chitosan and of the pentenoate proton signals at ~5.7, ~ 4.9, ~2.4 and ~2.2 ppm. DS was found equals to 0.2.

2.2.2. Chitosan-PEG synthesis

Chitosan-pentenoate was cross linked with SH-PEG-SH 3.4 kDalton polymer (Creative PEG Works) under UV light irradiation (Hamamatsu LC8 lamp, 365 nm irradiation with a power of 60 mW/cm$^2$ for 15 minutes or a power of 20 mW/cm$^2$ for 43 minutes) in the presence of Irgacure 2959 (Ciba) and with or without F50 nanoparticles (either neutral, anionic or cationic) at a 10% w/w or a 50% w/w nanoparticle/chitosan payload. For this purpose, the different solutions listed in Table 6 were prepared. 140 µL of the solutions were then poured into cylindrical PTE molds (0.5 mm high, 6 mm diameter) and placed under the UV lamp.

Table 6: Composition of solutions prepared for the synthesis of chitosan-PEG hydrogels including F50 nanoemulsions.

| Hydrogel | Chitosan-pentenoate (mg) | SH-PEG-SH (mg) | Irgacure 2959 (mg) | Nanoparticles (mg) | $CH_3COOH$ 0.3 M / $CH_3COONa$ 0.2 M (mL) |
|---|---|---|---|---|---|
| Chitosan-PEG | 30 | 20.52 | 1 | - | 1 |

(continued)

| Hydrogel | Chitosan-pentenoate (mg) | SH-PEG-SH (mg) | Irgacure 2959 (mg) | Nanoparticles (mg) | $CH_3COOH$ 0.3 M / $CH_3COONa$ 0.2 M (mL) |
|---|---|---|---|---|---|
| Chitosan-PEG encapsulating 10% w/w of "neutral" F50 | 30 | 20.52 | 1 | 3 "neutral" F50 of expl 1.1 (in 30 µL 1XPBS) | 0.970 |
| Chitosan-PEG encapsulating 50% w/w of "neutral" F50 | 30 | 20.52 | 1 | 15 "neutral" F50 of expl 1.1 (in 150 µL 1XPBS) | 0.850 |
| Chitosan-PEG encapsulating 10% w/w of "anionic" F50 | 30 | 20.52 | 1 | 3 "anionic" F50 of expl 1.1 (in 30 µL 1XPBS) | 0.970 |
| Chitosan-PEG encapsulating 50% w/w of "anionic" F50 | 30 | 20.52 | 1 | 15 "anionic" F50 of expl 1.1 (in 150 µL 1XPBS) | 0.850 |
| Chitosan-PEG encapsulating 10% w/w of "cationic" F50 | 30 | 20.52 | 1 | 3 "cationic" F50 of expl 1.1 (in 30 µL 1XPBS) | 0.970 |
| Chitosan-PEG encapsulating 50% w/w of "cationic" F50 | 30 | 20.52 | 1 | 15 "cationic" F50 of expl 1.1 (in 150 µL 1XPBS) | 0.850 |

2.3. Characterizations

The chitosan-PEG hydrogels encapsulating F50 nanoemulsions obtained in example 2.2 were characterized for their mechanical and water-swelling properties.

2.3.1 Mechanical properties

An AR2000Ex rheometer (TA Instruments, Inc.) fitted with a UV-curing cell ($\lambda$ = 365 nm) and an aluminum plate (diameter 20 mm) was used for the in situ measurement of the viscoelastic properties of the gels. On each hydrogel, oscillatory time sweep and frequency sweep experiments were performed. In the oscillatory time sweep experiments, the storage modulus (G') and loss modulus (G") were measured for a period of 43 min at a fixed frequency of 1 Hz and a fixed deformation of 3.5%. Typically, after deposition of the solution gel between the plates and equilibration for 1.5 min, the solution was illuminated $\lambda$ = 365 nm) for 43 min at a fixed light power (20 mW/cm$^2$) leading to gelation. All measurements were done in triplicates.

G' and G" plateaued after 10 minutes of irradiation, and the plateau values obtained after 30 minutes irradiation are listed in Table 7 for the different hydrogels.

Table 7: G' and G" of chitosan-PEG hydrogels including F50 nanoemulsions.

| Hydrogel | G' (Pa) |
|---|---|
| Chitosan-PEG | 12,510 ± 120 |
| Chitosan-PEG encapsulating 10% w/w of "neutral" F50 | 12,445 ± 205 |
| Chitosan-PEG encapsulating 50% w/w of "neutral" F50 | 11,285 ± 560 |
| Chitosan-PEG encapsulating 10% w/w of "anionic" F50 | 12,530 ± 105 |
| Chitosan-PEG encapsulating 50% w/w of "anionic" F50 | 12,270 ± 84 |
| Chitosan-PEG encapsulating 10% w/w of "cationic" F50 | 13,785 ± 20 |
| Chitosan-PEG encapsulating 50% w/w of "cationic" F50 | 11,280 ± 20 |

We observed that even for very high payload in nanoparticles, the mechanical properties of the hydrogels were preserved (10 % variation).

2.3.2 Swelling ratios

Swelling ratios of the hydrogels encapsulating F50 nanoemulsions obtained in example 2.2 were measured. For these purpose, hydrogels as obtained as described in example 2.2.2 were poured for 12 h in 10 mL of acetic acid 0.3 M / sodium acetate 0.2 M. The solution was removed and replaced by fresh acetic acid 0.3 M / sodium acetate 0.2 M. After 12 h the swelling material was put on a filter paper for 1 min to absorb excess of water then weight to obtain the swollen weight of the material in acetic acid 0.3 M / sodium acetate 0.2 M. The swollen material was then put into 10 mL of fresh deionized water for 12 hours. The material was weighted following the same protocol to obtain the swollen weight of the material in deionized water. Subsequently the polymer was dried in an oven at 50°C for 12 h to obtain the dried weight of polymer. It was checked that total drying was completed in 12 h.

The swelling ratio defined as SR = 100 x ($W_w$ - $W_d$) / $W_d$ was then calculated and the results are listed in Table 8.

Table 8: Swelling ratios of chitosan-PEG hydrogels including F50 nanoemulsions.

| Hydrogel | SR in water | SR in acetic acid 0.3 M / sodium acetate 0.2 M |
|---|---|---|
| Chitosan-PEG | 5310 ± 100 | 2870 ± 230 |
| Chitosan-PEG encapsulating 10% w/w of "neutral" F50 | 4960 ± 200 | 2610 ± 200 |
| Chitosan-PEG encapsulating 50% w/w of "neutral" F50 | 4760 ± 100 | 2310 ± 10 |
| Chitosan-PEG encapsulating 10% w/w of "anionic" F50 | 5040 ± 90 | 2210 ± 390 |
| Chitosan-PEG encapsulating 50% w/w of "anionic" F50 | 4900 ± 100 | 2270 ± 280 |
| Chitosan-PEG encapsulating 10% w/w of "cationic" F50 | 4960 ± 100 | 2440 ± 70 |
| Chitosan-PEG encapsulating 50% w/w of "cationic" F50 | 4910 ± 50 | 2370 ± 40 |

We observed that in the presence of the nanoparticles, the swelling ratio of the hydrogel was slightly decreased. However, even for very high payload in nanoparticles (50% w/w nanoparticle/chitosan), the swelling ratios of the hydrogels was not reduced by more than 25 %.

**Example 3: nanoparticles loaded in CMC-PEG hydrogels**

3.1. Preparation of F50 nanoemulsions

Same nanoparticles as example 1.1.

3.2. Preparation of CMC-PEG hydrogels including F50 nanoemulsions

CMC-PEG hydrogels (CMC: carboxy-methyl cellulose) including or not F50 nanoparticles (either neutral, anionic or cationic) at a 10% w/w or a 50% w/w nanoparticle/CMC payload were prepared by cross-linking under UV light in the presence of a photoinitiator (Irgacure 2959), CMC modified by alkene groups and a SH-PEG-SH polymer, according to Scheme I of example 2.2.

3.2.1. Synthesis of CMC-pentonoate

Alkene groups were introduced on CMC ("Blanose Cellulose Gum", Hercules, 100 kDalton) by reacting 4-pentenoïc anhydride with the polysaccharide, according to Scheme III.

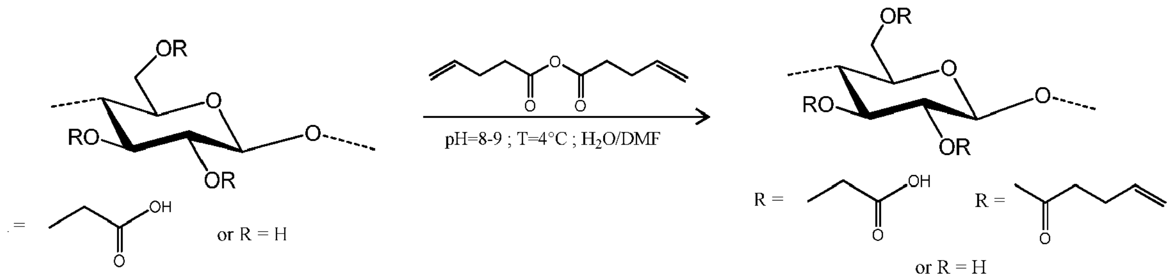


Scheme III. Chemical modification of CMC.

First, CMC (0.5 g) was dissolved in 25 mL of deionized water and stirred at 4°C overnight. 17 mL of DMF were added and the solution stirred for 2 hours at 4°C. 1.65 mL (1.65 mg, 4 eq.) of 4-pentenoic anhydride (Sigma-Aldrich) were diluted in 1 mL DMF then added dropwise to the CMC solution maintained at 4°C under stirring and at pH 8 by regular addition of NaOH 1 M. The mixture was then reacted for 4 hours while maintaining pH at 8 by regular addition of NaOH 1 M and stored overnight at 4°C. The modified polymer solution was then purified by ultrafiltration against deionized water using a 10 kDa cellulose membrane (filtration ultra membrane Amicon YM100 ultracell 10kDa). The polymer solution was then frozen in liquid nitrogen and lyophilized for 12 h (Labconbo freezone 6 lyophilisator, P = 0.05 mBar, T = -50°C) to obtain a white powder.

$^1$H NMR spectra of the CMC-pentenoate dissolved in deuterium oxide (6mg/mL) were performed at 80 °C using a Bruker DRX400 spectrometer operating at 400 MHz. All spectra were recorded by applying a 45° tip angle for the excitation pulse, and a 10 s recycle delay for 16 scans. The degree of substitution of the samples was determined by digital integration of the anomeric protons signals of CMC and of the pentenoate proton signals at ~5.7, ~ 4.9, ~2.4 and ~2.2 ppm. It is found equals to 0.2.

3.2.2. CMC-PEG synthesis

CMC-pentenoate was cross linked with SH-PEG-SH 3.4 kDalton polymer (Creative PEG Works) under UV light irradiation (Hamamatsu LC8 lamp, 365 nm irradiation with a power of 60 mW/cm$^2$ for 15 minutes or a power of 20 mW/cm$^2$ for 43 minutes) in the presence of Irgacure 2959 (Ciba) and including or not F50 nanoparticles (either neutral, anionic or cationic) at a 10% w/w or a 50% w/w nanoparticle/CMC payload to achieve CMC-PEG hydrogels. For this purpose, the different solutions listed in Table 9 for which the R=ene/thiol ratio (number of pentenoate groups of the CMC polymers over the number of thiols of the SH-PEG-SH polymers) was equal to 0.5 were prepared. 140 µL of the solutions was then poured into cylindrical PTE molds (0.5 mm high, 6 mm diameter) and placed under the UV lamp.

Table 9: Composition of solutions prepared for the synthesis of CMC-PEG hydrogels with R = 0.5 including F50 nanoemulsions.

| Hydrogel | CMC-pentenoate [mg] | SH-PEG-SH [mg] | Irgacure 2959 [mg] | Nanoparticles [mg] | PBS 1X [mL] |
|---|---|---|---|---|---|
| CMC-PEG R = 0.5 | 30 | 18.4 | 1 | - | 1 |
| CMC-PEG R = 0.5 encapsulating 10% w/w of "neutral" F50 | 30 | 18.4 | 1 | 3 "neutral" F50 prepared in example 1.1 (in 30 µL 1X PBS) | 0.970 |
| CMC-PEG R = 0.5 encapsulating 50% w/w of "neutral" F50 | 30 | 18.4 | 1 | 15 "neutral" F50 prepared in example 1.1 (in 150 µL 1X PBS) | 0.850 |
| CMC-PEG R = 0.5 encapsulating 10% w/w of "anionic" F50 | 30 | 18.4 | 1 | 3 "anionic" F50 prepared in example 1.1 (in 30 µL 1X PBS) | 0.970 |
| CMC-PEG R = 0.5 encapsulating 50% w/w of "anionic" F50 | 30 | 18.4 | 1 | 15 "anionic" F50 prepared in example 1.1 (in 150 µL 1X PBS) | 0.850 |

(continued)

| Hydrogel | CMC-pentenoate [mg] | SH-PEG-SH [mg] | Irgacure 2959 [mg] | Nanoparticles [mg] | PBS 1X [mL] |
|---|---|---|---|---|---|
| CMC-PEG R = 0.5 encapsulating 10% w/w of "cationic" F50 | 30 | 18.4 | 1 | 3 "cationic" F50 prepared in example 1.1 (in 30 μL 1X PBS) | 0.970 |
| CMC-PEG R = 0.5 encapsulating 50% w/w of "cationic" F50 | 30 | 18.4 | 1 | 15 "cationic" F50 prepared in example 1.1 (in 150 μL 1X PBS) | 0.850 |

CMC-PEG hydrogels encapsulating 50% w/w of "neutral" F50 nanoparticles (particle/CMC payload) were also prepared with different ene/thiol ratios to achieve hydrogels with different cross-link density. For this purpose, the different solutions listed in Table 10 were prepared. 140 μL of the solutions were then poured into cylindrical PTE molds (0.5 mm high, 6 mm diameter) and placed under the UV lamp (Hamamatsu LC8 lamp, 365 nm irradiation with a power of 60 mW/cm$^2$ for 15 minutes or a power of 20 mW/cm$^2$ for 43 minutes).

Table 10: Composition of solutions prepared for the synthesis of CMC-PEG hydrogels including 50% w/w of "neutral" F50 nanoemulsions.

| Hydrogel | CMC-pentenoate [mg] | SH-PEG-SH [mg] | Irgacure 2959 [mg] | Nanoparticles | PBS1X [mL] |
|---|---|---|---|---|---|
| CMC-PEG R = 0.3 | 30 | 12.6 | 1 | - | 1 |
| CMC-PEG R = 0.3 encapsulating 50% w/w of "neutral" F50 | 30 | 12.6 | 1 | 15 "neutral" F50 of expl 1.1 (in 150 μL 1X PBS) | 0.850 |
| CMC-PEG R = 0.5 | 30 | 18.4 | 1 | - | 1 |
| CMC-PEG R = 0.5 encapsulating 50% w/w of "neutral" F50 | 30 | 18.4 | 1 | 15 "neutral" F50 of expl 1.1 (in 150 μL 1X PBS) | 0.850 |
| CMC-PEG R = 1.0 | 30 | 44.1 | 1 | - | 1 |
| CMC-PEG R = 1.0 encapsulating 50% w/w of "neutral" F50 | 30 | 44.1 | 1 | 15 "neutral" F50 of expl 1.1 (in 150 μL 1X PBS) | 0.850 |

3.3. Characterizations

The CMC-PEG hydrogels encapsulating F50 nanoemulsions obtained in example 3.2 were characterized for their mechanical and water-swelling properties.

3.3.1 Mechanical properties

An AR2000Ex rheometer (TA Instruments, Inc.) fitted with a UV-curing cell (λ = 365 nm) and an aluminum plate (diameter 20 mm) was used for the in situ measurement of the viscoelastic properties of the gels. On each hydrogel, oscillatory time sweep and frequency sweep experiments were performed. In the oscillatory time sweep experiments, the storage modulus (G') and loss modulus (G") were measured for a period of 43 min at a fixed frequency of 1 Hz and a fixed deformation of 3.5%. Typically, after deposition of the solution gel between the plates and equilibration for 1.5 min, the solution was illuminated λ = 365 nm) for 43 min at a fixed light power (20 mW/cm$^2$) leading to gelation. Light illumination was then stopped for 1.5 min. All measurements were done in triplicates.

G' and G" plateaued after 10 minutes of irradiation, and the plateau values obtained after 30 minutes irradiation are listed respectively in Table 11 and Table 12 for the different hydrogels issued from the UV-induced cross-linking of solutions listed in Table 9 and Table 10 respectively obtained as described in example 3.2.

Table 11: G' and G" (shearing test) of CMC-PEG hydrogels including F50 nanoemulsions issued from the UV-induced cross-linking of solutions listed in Table 9 (R = 0.5).

| Hydrogel | G' (Pa) |
|---|---|
| CMC-PEG R = 0.5 | 11,750,270 ± 520 |
| CMC-PEG R = 0.5 encapsulating 10% w/w of "neutral" F50 | 12,655 ± 305 |
| CMC-PEG R = 0.5 encapsulating 50% w/w of "neutral" F50 | 11,245 ± 100 |
| CMC-PEG R = 0.5 encapsulating 10% w/w of "anionic" F50 | 12,455 ± 260 |
| CMC-PEG R = 0.5 encapsulating 50% w/w of "anionic" F50 | 11,220 ± 210 |
| CMC-PEG R = 0.5 encapsulating 10% w/w of "cationic" F50 | 12,295 ± 205 |
| CMC-PEG R = 0.5 encapsulating 50% w/w of "cationic" F50 | 10,970 ± 155 |

Table 12: G' and G" (shearing test) of CMC-PEG hydrogels including 50% w/w of "neutral" F50 nanoemulsions issued from the UV-induced cross-linking of solutions listed in Table 10.

| Hydrogel | G' (Pa) |
|---|---|
| CMC-PEG R = 0.3 | 5,640 ± 140 |
| CMC-PEG R = 0.3 encapsulating 50% w/w of "neutral" F50 | - |
| CMC-PEG R = 0.5 | 11,750 ± 520 |
| CMC-PEG R = 0.5 encapsulating 50% w/w of "neutral" F50 | 11,345 ± 120 |
| CMC-PEG R = 1.0 | 21,430 ± 250 |
| CMC-PEG R = 1.0 encapsulating 50% w/w of "neutral" F50 | 21,840 ± 315 |

Varying the thiol-ene ratio (and hence the cross-link density) allowed the synthesis of hydrogels with different elastic properties: for higher R (higher cross-linking) the materials were more rigid (higher elastic moduli G') (Table 12). We observed that even for very high payload in nanoparticles (up to 50% w/w of nanoparticles considering the nanoparticle/polymer ratio), the mechanical properties of the materials considering shearing stress were not modified significantly (less than 15 %), whatever the nanoparticle charge (Tables 11 and 12).

The Young modulus E of the hydrogels were also measured by uniaxial compression tests performed on the different hydrogels issued from the UV-induced cross-linking of solutions listed in Table 9 and Table 10 respectively using an AR2000EX rheometer (TA instrument) with an initial preload of 0.03 N and a compression speed of 10 $\mu$m/s. The stress, $\sigma$, was calculated as the normal force divided by the surface area of the material (6 mm diameter), whereas the displacement data were expressed as an extension ratio, $\lambda = h/h0$, where h is the deformed height. The $\sigma = f(1-\lambda)$ plots obtained were typical of an elastic hydrogel material and the Young modulus E of the materials could be calculated as the slopes of $\sigma = f(1-\lambda)$ for low strains ($\lambda \rightarrow 1$, linear domain).

Results are listed respectively in Table 13 for the different hydrogels issued from the UV-induced cross-linking of solutions listed in Table 9 obtained as described in example 3.2. Data are presented as mean ± standard deviation of experiments performed on 6 different materials.

Table 13: E (compression test) of CMC-PEG hydrogels including F50 nanoemulsions issued from the UV-induced cross-linking of solutions listed in Table 9 (R = 0.5).

| Hydrogel | E (Pa) |
|---|---|
| CMC-PEG R = 0.5 | 118,100 ± 17,600 |
| CMC-PEG R = 0.5 encapsulating 10% w/w of "neutral" F50 | 111,000 ± 19,600 |
| CMC-PEG R = 0.5 encapsulating 50% w/w of "neutral" F50 | 158,100 ± 17,600 |
| CMC-PEG R = 0.5 encapsulating 10% w/w of "anionic" F50 | 165,000 ± 12,000 |
| CMC-PEG R = 0.5 encapsulating 50% w/w of "anionic" F50 | 139,900 ± 11,400 |

(continued)

| Hydrogel | E (Pa) |
|---|---|
| CMC-PEG R = 0.5 encapsulating 10% w/w of "cationic" F50 | 197,600 ± 17,900 |
| CMC-PEG R = 0.5 encapsulating 50% w/w of "cationic" F50 | 177,300 ± 9,900 |

Contrarily to the non-modification of the mechanical properties of the materials under shearing stress, the addition of nanoparticles improved the rigidity of the hydrogels during compression (increase of the Young modulus). The effect was more stressed for cationic nanoparticle payload, up to 70% increase in the Young modulus for 10% w/w cationic F50 (Table 13).

3.3.2 Swelling ratios

Swelling ratios of the hydrogels encapsulating F50 nanoemulsions obtained in example 3.2 were measured. For these purpose, hydrogels as obtained as described in example 3.2.2 were poured for 12 h in 10 mL of 1X PBS. The solution was then removed and replaced by fresh 1X PBS. After 12 h the swollen material was put on a filter paper for 1 min to absorb excess of water and then weight to obtain the swollen weight of the material in 1X PBS. The swollen material was then put into 10 mL of fresh deionized water for 12 hours. The material was weighted following the same protocol to obtain the swollen weight of the material in deionized water. Subsequently the polymer was dried in an oven at 50°C for 12 h to obtain the dried weight of polymer. It was checked that total drying was completed in 12 h.

The swelling ratio defined as SR = 100 x (Ww - Wd) / Wdwas then calculated and the results are listed in Table 14.

Table 14: Swelling ratios of CMC-PEG hydrogels including F50 nanoemulsions issued from the UV-induced cross-linking of solutions listed in Table 9 (R = 0.5).

| Hydrogel | SR in water | SR in 1X PBS |
|---|---|---|
| CMC-PEG R = 0.5 | 8140 ± 100 | 2810 ± 40 |
| CMC-PEG R = 0.5 encapsulating 10% w/w of "neutral" F50 | 6450 ± 280 | 2270 ± 100 |
| CMC-PEG R = 0.5 encapsulating 50% w/w of "neutral" F50 | 6370 ± 30 | 2140 ± 110 |
| CMC-PEG R = 0.5encapsulating 10% w/w of "anionic" F50 | 7270 ± 190 | 2790 ± 70 |
| CMC-PEG R = 0.5 encapsulating 50% w/w of "anionic" F50 | 6900 ± 90 | 2360 ± 10 |
| CMC-PEG R = 0.5 encapsulating 10% w/w of "cationic" F50 | 7740 ± 760 | 3010 ± 330 |
| CMC-PEG R = 0.5 encapsulating 50% w/w of "cationic" F50 | 6840 ± 560 | 2440 ± 40 |

We observed that in the presence of the nanoparticles, the swelling ratio of the hydrogels was slightly decreased. However, even for very high payload in nanoparticles (50% w/w nanoparticle/CMC), the swelling ratios of the hydrogels was not reduced by more than 25 %.

3.4. Release of nanoparticles from CMC-PEG hydrogels

The kinetics of release from the CMC-PEG hydrogels of the F50 nanoparticles (either "neutral", anionic or cationic) was assessed using the following protocol. Hydrogel materials prepared as described in example 3.2 were separated in 4 pieces of about 30 mg which were accurately weighted. Each piece was immersed in 1 mL of 1X PBS and stored in dark at ambient temperature. At the desired time point, the piece of material was taken and immersed in 1 mL of 0.1 M NaOH for 15 minutes to dissolve the hydrogel and release the nanoparticles remaining entrapped inside the material. 900 μL of the solution was then transferred in a quartz microcuvette for fluorescence and nanoparticle size analysis. The number of nanoparticles released from the material was quantified by fluorescence using the Dil fluorophore

encapsulated in the nanoparticle core, as described in example 1.1. Fluorescence titration was made using a Perkin Elmer LS50B fluorimeter using 520 nm excitation and a calibration curve allowed for the quantification of the number of released nanoparticles. Data were normalized by the exact weight of the piece of material, and data expressed in % of released nanoparticles are summarized in Table 15 and Table 17. Nanoparticle size and polydispersity index were also checked by Dynamic Light Scattering of the dispersion after its filtration on 0.22 μm filter to eliminate remaining parts of polymer (Zeta Sizer Nano ZS, Malvern Instrument). Results are summarized in Table 16 and Table 18.

Table 15: % of nanoparticles released from CMC-PEG hydrogels R = 0.5 including 10% w/w of F50 nanoemulsions.

| Time (days) | CMC-PEG R = 0.5 10% w/w of "neutral" F50 | CMC-PEG R = 0.5 10% w/w of anionic F50 | CMC-PEG R = 0.5 10% w/w of cationic F50 |
|---|---|---|---|
| 0 | 0 | 0 | 0 |
| 1 | -10.5±0.4 | 6.0±1.5 | 13.2 ± 1.6 |
| 5 | 40.1 ± 4.6 | 31.3 ±12.3 | 13.5 ± 1.8 |
| 7 | 32.0 ± 4.1 | 29.9 ± 3.2 | - |
| 15 | 40.6 ± 0.3 | 35.0 ± 13.05 | 17.7 ± 2.3 |
| 30 | 44.2 ± 1.5 | 46.6 ± 16.3 | 15.9 ± 1.4 |
| 45 | 48.1 ± 4.8 | 54.5 ± 9.3 | - |

Table 16: Size (polydispersity index) of nanoparticles entrapped within the CMC-PEG hydrogels R=0.5 including 10% w/w of F50 nanoemulsions, then released upon material degradation in NaOH.

| Time (days) | CMC-PEG R = 0.5 10% w/w of "neutral" F50 | CMC-PEG R = 0.5 10% w/w of anionic F50 | CMC-PEG R = 0.5 10% w/w of cationic F50 |
|---|---|---|---|
| 0 | 126 ±6 (0.29 ±0.01) | 132 ± 14 (0.29 ±0.01) | 173 ± 6.22 (0.30 ± 0.01) |
| 1 | 128 ±6 (0.29 ±0.01) | 135±5(0.28±0.02) | 163 ± 1 (0.30 ± 0.01) |
| 5 | 123 ± 2 (0.29 ± 0.01) | 133 ± 4 (0.28 ± 0.01) | 144 ±5 (0.28 ±0.01) |
| 7 | 122 ± 1 (0.29 ± 0.01) | 132 ± 1 (0.29 ± 0.01) | 138 ± 2 (0.28 ±0.01) |
| 15 | 130 ± 10 (0.30 ±0.01) | 118 ±5 (0.36 ±0.05) | |
| 30 | 123 ± 1 (0.30 ± 0.01) | 125 ± 12 (0.27 ±0.01) | |
| 45 | 91 ± 1 (0.21 ±0.01) | 123 ±4 (0.27 ±0.01) | |

Table 17: % of nanoparticles released from CMC-PEG hydrogels R = 0.5 including 50% w/w of F50 nanoemulsions.

| Time (days) | CMC-PEG R = 0.5 50% w/w of "neutral" F50 | CMC-PEG R = 0.5 50% w/w of anionic F50 |
|---|---|---|
| 0 | 0 | 0 |
| 1 | - | 0 |
| 5 | 23.3 ± 4.6 | 2.2 ± 0.08 |
| 7 | 17.9 ± 4.0 | 0.1 ± 0.03 |
| 15 | 22.4 ± 0.3 | 31.7 ± 1.04 |
| 30 | 8.9 ± 0.2 | 58.7 ± 3.4 |
| 45 | 10.6 ± 1.2 | 63.0 ± 14.5 |

Table 18: Size (polydispersity index) of nanoparticles released from CMC-PEG hydrogels R=0.5 including 50% w/w of F50 nanoemulsions.

| Time (days) | CMC-PEG R = 0.5 50% w/w of "neutral" F50 | CMC-PEG R = 0.5 50% w/w of anionic F50 |
|---|---|---|
| 0 | 95 ±5 (0.26 ±0.01) | 120 ± 35 (0.34 ± 0.08) |
| 1 | 96 ±5 (0.26 ±0.01) | 89 ± 4 (0.28 ± 0.05) |
| 5 | 84 ±15 (0.26 ±0.01) | 100 ± 8 (0.25 ± 0.01) |
| 7 | 80 ±7 (0.26 ±0.01) | 86 ± 2 (0.24 ± 0.01) |
| 15 | 93 ±5 (0.26 ±0.01) | 100 ± 8 (0.24 ± 0.01) |
| 30 | 92 ±2 (0.25 ±0.01) | 92 ± 4 (0.20 ± 0.01) |
| 45 | 91 ± 1 (0.16 ± 0.01) | 97 ± 3 (0.21 ±0.01) |

Nanoparticles entrapped then released from the CMC-PEG materials were bigger than their initial size and their dispersion displayed an increased polydispersity, since after their inclusion then released from the hydrogel after its degradation, some polymer chain were probably coating the nanoparticle surface, accounting for their increase of size and polydispersity (Tables 16 and 18). However, we observed that the diameter and PDI of the extracted nanoparticles from the hydrogel were constant with incubation time, indicating a good colloidal stability of the nanoparticles when embedded in the swollen material even for days.

We observed different kinetics of release according to the nanoparticle charge and size: bigger cationic nanoparticles were released far more slowly than smaller neutral nanoparticles, which were also released more slowly than anionic nanoparticles (Tables 15 and 17). The faster release of anionic nanoparticles could be accounted for by the negative charge of the CMC matrix at pH 7.4 (PBS buffer), that could favor the electrostatic repulsion between the scaffold and the anionic nanoparticles and enhance their leakage.

<u>**Example 4: nanoparticles loaded in collagen** material</u>

<u>4.1. Preparation of F50 and F120</u>

F50 and F120 emulsions with the composition detailed in Table 19 were prepared. An oily premix was prepared by mixing refined soybean oil, Suppocire NC™ and Lipoid S75™. After homogeneization at 50°C and complete evaporation of ethanol, the continuous aqueous phase, composed of Myrjs40™ and of aqueous medium was introduced. The vial was placed in a 50°C water bath and the mixture was sonicated for 5 min using a VCX750 ultrasonic processor (power output 190 W, 3-mm probe diameter, Sonics). The obtained emulsions were dialyzed overnight at room temperature (about 20°C) (20°C) against 1000 times their volume in the appropriate aqueous buffer (12-14,000 Da MW cut off membranes, ZelluTrans). Finally, the concentration of the emulsion was adjusted to 100 mg/mL (10% w/w) of lipids in 1X PBS (lipids include oil + Suppocire + Lipoid + Myrjs40), and filtered through a 0.22 μm Millipore membrane (F50) or through a 0.45 μm Millipore cellulose membrane (F120).

Table 19: Composition of the F50 and F120 emulsions.

| Compound | Provider | Quantity (mg) | |
|---|---|---|---|
| | | F50 | F120 |
| refined soybean oil | Croda Uniqema, France | 85 | 150 |
| Suppocire NC™ | Gatefosse, France | 255 | 450 |
| Lipoid S75™ | Lipoid, Germany | 65 | 45 |
| Myrjs40™ | Croda Uniqema, France | 345 | 215 |
| aqueous medium: 1X PBS | | qsp 2 ml | qsp 2 ml |

The emulsions were characterized by their size and polydispersity index (PdI), using Dynamic Light Scattering (Zeta Sizer Nano ZS, Malvern Instrument). For this purpose, the emulsions were diluted to achieve 2 mg/ml of components

of the nanoparticles (ie: combination of oil + Suppocire + Lipoid + Myrjs40) in sterile PBS 0.1X and were transferred in Zeta Sizer Nano cells (Malvern Instrument) before each measurement, performed in triplicate.

Results are described in Table 20.

Table 20. Nanoparticle diameter and PdI of the prepared emulsions

| | Nanoparticles concentration (mg/mL) | Nanoparticle diameter (nm) | Polydispersity Index (PdI) |
|---|---|---|---|
| F50 | 100 | 60 ± 1 | 0.19 ± 0.01 |
| F120 | 100 | 120 ±2 | 0.15 ± 0.01 |

The emulsions were stable more than 5 months at 4°C (the nanoparticle diameters and PdI thereof did not change).

4.2. (Collagen / nanoparticles) material from the emulsions prepared

The F50 and F120 emulsions prepared in example 4.1 were loaded in a collagen gel (Type I collagen from equine Achilles tendon, prepared at 1% w/v in acetic acid pH 3, Euroresearch) at a final weight ratio of dry collagen/nanoparticles 10/1 and lyophilized. The directional freeze-drying procedure was carried out to provide a sponge like material with a uniform porous microstructure. Either 3 g of gel material were distributed into a 35 mm Petri dish (FALCON Easy Grip Tissue Culture Dish, 353001), or 2 g of gel material were distributed into each well of 12 Well Cell Culture Plate. The air or upper surface was covered with a polypropylene membrane before the freezing. The Petri dish and the 12 Well Plate were placed into a glass container and quickly frozen in liquid nitrogen for 5 min. The freeze-drying process was done for 24 hours with a Cryotec V8.11 plate lyophilizer. After freeze-drying process the polypropylene membrane was removed with a tweezer. Finally, a lyophilized white material was obtained, with composition of 1 mg of nanoparticles for 10 mg of dry collagen.

Before subsequent utilization, the lyophilized material can be hydrated by incubation in the appropriate aqueous buffer (water, 1X PBS, cell culture medium...) to yield a hydrogel containing the F50 or F120 nanoparticles. The material does not disintegrate for several days after its incubation at room temperature in water or 1X PBS (about 5 mL).

4.3. Mechanical properties of the (Collagen / nanoparticles) materials

The mechanical properties of the (collagen/nanoparticles 10/1) hydrogels obtained after 24h incubation of the materials synthetized as described in example 4.2 in 5 mL of water, then quick drying of the over excess of liquid on a filter paper were assessed both their shearing and compression properties. The properties of the hydrogels were compared to those of a hydrogel composed of 100% of collagen and prepared according to the same protocol (lyophilized material then rehydrated in 5 mL of water).

Measurements were performed on the pieces of hydrogels (collagen, collagen/F50 10/1, collagen/F120 10/1) using an AR2000EX rheometer (TA instrument) with an initial preload of 0.03 N and a compression speed of 10 $\mu$m/s. The stress, $\sigma$, was calculated as the normal force divided by the surface area of the material, whereas the strain, $\varepsilon$, was the % of compression of the material. Stress-strain curves were plotted as $\sigma_{true}$=f($\lambda$'), where $\lambda' = 1 - \varepsilon$ and $\sigma_{true} = \sigma \times \lambda'$. From this curve, the Young modulus E of the hydrogels can be deduced from the slope in the Hookean (linear) regime. The elastic modulus G' can be deduced from the plot of $\sigma_{red}$=f(1/$\lambda$'), where

$$\sigma_{red} = \frac{-\sigma}{l' - \frac{1}{l'^2}}$$

(W.-C. Lin, W. Fan, A. Marcellan, D. Hourdet, C. Creton. "Large Strain and Fracture Properties of Poly(dimethylacrylamide)/Silica Hybrid Hydrogels". Macromolecules, vol.43, pp.2554-2563, 2010.)

Values are presented in Table 21.

Table 21. Young modulus E and elastic moduli G' for the materials.

| | E [Pa] | G' [Pa] |
|---|---|---|
| Collagen | 373 ± 24 | 98_±9 |
| Collagen/F50 10/1 | 239 ± 60 | 85 ± 10 |

(continued)

| | E [Pa] | G' [Pa] |
|---|---|---|
| Collagen/F120 10/1 | 230 ± 80 | 100 ± 24 |

We observed that whereas the elastic modulus G' was slightly impacted by the nanoparticle payload, a more important effect was observed on the Young modulus E of the hydrogels (decreased by 36 %).

**Example 5: Curcumin-loaded nanoparticles loaded in collagen material**

5.1. Preparation of F50, F80 and F120 emulsions the nanoparticles of which are loaded with curcumin

Curcumin is a polyphenol widely used in traditional medicines in China and India for centuries. It has strong antioxidant, antiseptic and anti-inflammatory properties, as well as potentially MMP inhibiting activity, and has aroused interest in numerous animal studies on wound healing. The very low solubility of curcumin in aqueous solvents (0.4 μg/ml at pH 7.3) and its very fast degradation at pH > 6.5 make necessary the development of dedicated formulations for curcumin administration.

F50, F80 and F120 emulsions the nanoparticles of which were loaded with curcumin were prepared, with an initial payload of 1% wt of curcumin compared to the weight of nanoparticles, as indicated in the composition detailed in Table 22. An oily premix was prepared by mixing refined soybean oil, Suppocire NC™, Lipoid S75™ and curcumin. After homogeneization at 50°C and complete evaporation of ethanol, the continuous aqueous phase, composed of Myrjs40™ and of aqueous medium was introduced. The vial was placed in a 50°C water bath and the mixture was sonicated for 5 min using a VCX750 ultrasonic processor (power output 190 W, 3-mm probe diameter, Sonics). The obtained emulsions were dialyzed overnight at room temperature (about 20°C) (20°C) against 1000 times their volume in the appropriate aqueous buffer (12-14,000 Da MW cut off membranes, ZelluTrans). Finally, the concentration of the emulsion was adjusted to 100 mg/mL (10% w/w) of lipids in 1X PBS (lipids include oil + Suppocire + Lipoid + Myrjs40), and filtered through a 0.22 μm Millipore membrane F50 and F80) or through a 0.45 μm Millipore cellulose membrane.

Table 22: Composition of the curcumin emulsions.

| Compound | Provider | Quantity (mg) | | |
|---|---|---|---|---|
| | | F50-curcumin | F80-curcumin | F120-curcumin |
| refined soybean oil | Croda Uniqema, France | 85 | 102.5 | 150 |
| Suppocire NC™ | Gatefosse, France | 255 | 307.5 | 450 |
| Curcumin | Sigma-Aldrich | 7.5 | 7.6 | 8.6 |
| Lipoid S75™ | Lipoid, Germany | 65 | 50 | 45 |
| Myrjs40™ | Croda Uniqema, France | 345 | 300 | 215 |
| aqueous medium: 1X PBS | | qsp 2 ml | qsp 2 ml | qsp 2 ml |

It is recommended to extract the curcumin from the emulsions first in order to avoid injecting the lipid components which would absorb irreversibly on the stationary phase of the HPLC column. This extraction was carried out in four steps using two different solvents:

Solvent A: HCl 0.1 N + acetonitrile (10%- 90%);
Solvent B : acetonitrile + acetic acid diluted in water at 2% (60% - 40%).

1) The samples to be analyzed were diluted in PBS (phosphate and NaCl at pH 7.4) to obtain:

- a maximal lipid concentration of 60 mg/ml ;
- a maximal curcumin concentration of 0.6 mg/ml.

2) The nanoparticles were destroyed by diluting the samples in 100 μl of solvent A, the curcumin concentration being then less than or equal to 300 μg/ml. Then, the samples were homogenized by vortex mixing.
3) The lipids were eliminated by precipitation. More precisely, the samples were diluted in 300 μl of de solvent B.

(The curcumin concentration being then less than or equal to 120 μg/ml). After vortex mixing followed by 10 min of centrifugation at 10 000 rpm, a precipitate containing the lipids was obtained.
4) 100 μl of the supernatant were collected (comprising less than or equal to 12 μg of curcumin) and diluted in 900 μl of solvent B. The curcumin concentration was less than or equal to 12 μg/ml.

In order to determine the curcumin concentration, a reverse phase column composed of silica grafted with linear chains comprising 18 carbon atoms (C18 SunFire®), which is a non-polar stationary phase, was used.
The eluant was a mixture of acetonitrile and of acetic acid diluted in water at 2% v/v the proportions of which changed according to a gradient. During the first two minutes: 40% of diluted acetic acid and 60% of acetonitrile, then a linear gradient the 10 following minutes until 100% of acetonitrile, the 5 following minutes a linear gradient to come back to 40% of diluted acetic acid and 60% of acetonitrile, and the last 3 minutes with 40% of diluted acetic acid and 60% of acetonitrile.
Detection was performed by UV absorption at a wavelenght $\lambda$ = 420 nm. With this procedure, the chromatographic peak of curcumin appeared at t = 6 min, i.e. at about 75% of acetonitrile and 25% of diluted acetic acid.
A calibration curve was obtained from samples of known concentrations and used for the determination of the concentration in curcumin. The curcumin encapsulation yields were of 51 ± 2 % for the curcumin F50 emulsion, of 74 ± 3 % for the curcumin F80 emulsion and 92 ± 4 % for the curcumin F120 emulsion.
The emulsions were characterized by their size and polydispersity index (PdI), using Dynamic Light Scattering (Zeta Sizer Nano ZS, Malvern Instrument). For this purpose, the emulsions were diluted to achieve 2 mg/ml of components of the nanoparticles (ie: combination of oil + Suppocire + Lipoid + Myrjs40) in sterile PBS 0.1X and were transferred in Zeta Sizer Nano cells (Malvern Instrument) before each measurement, performed in triplicate.
Results are described in Table 23.

Table 23. Nanoparticle diameter and PdI of the prepared emulsions

| Emulsion | Nanoparticles concentration (mg/mL) | Nanoparticle diameter (nm) | Polydispersity Index (PdI) |
|---|---|---|---|
| curcumin-F50 | 100 | 60 ± 1 | 0.19 ± 0.01 |
| curcumin-F80 | 100 | 79 ± 1 | 0.16 ± 0.01 |
| curcumin-F120 | 100 | 120 ±2 | 0.15 ± 0.01 |

The curcumin emulsions were stable more than 5 months at 4°C (the nanoparticle diameters and PdI thereof did not change).

<u>5.2. (Collagen / nanoparticles comprising curcumin) material from the emulsions prepared</u>

The F50, F80 and F120 emulsions comprising curcumin prepared in example 5.1 were loaded in a collagen gel (Type I collagen from equine Achilles tendon, prepared at 1% w/v in acetic acid pH 3, Euroresearch) at a final weight ratio of dry collagen/nanoparticles 10/1 and lyophilized. The directional freeze-drying procedure was carried out to provide a sponge like material with a uniform porous microstructure. Either 3 g of gel material were distributed into a 35 mm Petri dish (FALCON Easy Grip Tissue Culture Dish, 353001), or 2 g of gel material were distributed into each well of 12 Well Cell Culture Plate. The air or upper surface was covered with a polypropylene membrane before the freezing. The Petri dish and the 12 Well Plate were placed into a glass container and quickly frozen in liquid nitrogen for 5 min. The freeze-drying process was done for 24 hours with a Cryotec V8.11 plate lyophilizer. After freeze-drying process the polypropylene membrane was removed with a tweezer. Finally, a lyophilized pale yellow material was obtained, with composition of 10 μg of curcumin and 1 mg of nanoparticles for 10 mg of dry collagen.
For comparison, curcumin was directly included in the collagen material. For this purpose, 30 μg of curcumin in solution in ethanol (2 mg/mL) was mixed with 3 g of collagen gel at 1% w:w in aqueous acetic acid. The mixture was homogeneized by stirring at room temperature (about 20°C) (20°C) for a few minutes, then the material was lyophilized as previously described. Finally, a lyophilized pale yellow material (30 mg) was obtained with 10 μg of curcumin for 10 mg of dry material.

<u>5.3. Structural characterization of the material obtained</u>

Figures 2 to 13 and 14 to 21 are SEM and TEM images of the obtained (collagen / nanoparticles comprising curcumin) material and of the collagen material comprising curcumin but free of nanoparticles (control material).
The morphology and porosity of the material obtained were explored by Scanning Electron Microscopy (SEM).

To this end, the materials were cut with a sterile razor blade into a number of small blocks and attached to aluminum stubs with a carbon tape. Blocks were oriented in such a way as to expose either the upper or the lower surfaces, while others were glued on their longitudinal cut profile. They were sputter-coated with gold in a Balzers MED 010 unit and observed in a JEOL JSM 6010LA electron microscope.

The ultrastructure of the obtained materials was explored by Transmission Electron Microscopy (TEM). The materials were cut into a number of small blocks with a sterile razor blade and fixed overnight at 4 °C with 3% glutaraldehyde in phosphate buffer (PB) at pH 7.2. Following extensive rinsing with the same buffer at 4 °C, they were immersed for 1h in 0.5 % tannic acid in PB at 4 °C. They were then rinsed again four times in the same buffer for 15 min at 4 °C, and post-fixed for 1h with 1% osmium tetroxide in PB at 4 °C. Specimens were finally washed in distilled water, block-stained with 1% uranyl acetate in distilled water and then dehydrated in a graded series of ethanol.The dehydrated samples were infiltrated for two days with graded mixtures of LRWhite resin/ethanol. By the end of this procedure, samples were embedded in fresh LRWhite resin and cut with Reichert Ultracut ultramicrotome equipped with a diamond knife. Ultrathin sections (60-80 nm thick) were collected on copper grids, stained with uranyl acetate and lead citrate, and observed with a JEOL 1200 EX II electron microscope. Micrographs were acquired by the Olympus SIS VELETA CCD camera equipped the iTEM software.

Figures 2 to 13 demonstrate that direct loading of curcumin in the collagen matrix can induce some clotting of the water-insoluble therapeutic agent and block the material pores (especially visible for the lower and upper surface of the material). On the contrary, similar payload without any structure alteration can be achieved with the (collagen/nanoparticles comprising curcumin) materials.

Figures 14 to 21 demonstrate that direct loading of curcumin in the collagen matrix (control) leads to more fibrillar structures, whereas good organization of the collagen fibers is evidenced with the (collagen/nanoparticles comprising curcumin) materials.

In conclusion, therapeutic agent loading in the collagen material through the use of the nanoparticles allows preservation of the micro- and ultra-structure of the material, in comparison to direct therapeutic agent loading.

**Example 6: Growth-factor functionalized nanoparticles loaded in collagen material**

Human platelet-derived growth factor fragment BB (PDGF-BB), a 25 kDalton protein, is the most cited growth factor derivative that has been investigated for wound healing promoting properties. This growth factor was therefore covalently linked to F120 nanoparticles functionalized by a maleimide group, to be further inserted in the collagen matrix.

In order to achieve F120 nanoparticles functionalized by a maleimide group, a functionalizable surfactant, i.e. a stearate-PEG surfactant bearing at the poly(ethyleneglycol) extremity a maleimide group, was first synthetized, then introduced at the nanoparticle surface during formulation. The maleimide-F120 nanoparticles were then covalently linked to PDGF-BB.

6.1. Synthesis of the stearate-PEG-maleimide surfactant $SA_{CONH}$ -$PEG_{100}$-mal

The stearate-PEG-maleimide surfactant of formula I was synthetized as described below. All chemical products were purchased from Sigma-Aldrich (Saint-Quentin Fallavier, France) and used as such, unless specified. 1 H NMR spectra were measured on a Bruker 300 spectrometer in deuterated chloroform ($CDCl_3$). Peaks were referenced against residual solvent at 7.26 ppm ($CDCl_3$).

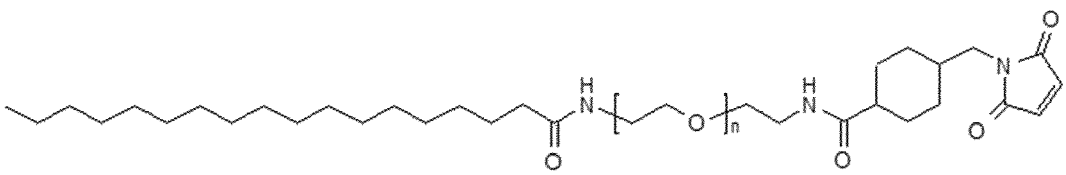

n = 100

Formula I

*Synthesis of $SA_{CONH}$ -$PEG_{100}$-NHBoc*

An amount of 1.03 g (3.6 mmol) of stearic acid and 1.6 g (3.6 mmol) of (benzotriazol-1-yloxy)tris(dimethylamino)phosphonium hexafluorophosphate (BOP) were added to 80 mL of DCM until complete dissolution. 5 g of BocNH-$PEG_{100}$-$NH_2$ (1.03 mmol, Iris Biotech, Marktredwitz, Germany) and 470 mg of N,N-Diisopropylethylamine (DIEA) (3.6 mmol) were then added and the mixture was stirred for two hours at room temperature (about 20°C). Solvent was then

reduced under reduced pressure and precipitation was obtained upon addition of cold diethyl ether. The precipitate was filtered, dissolved in water, extensively dialysed against water (MWCO 1 kDa) and lyophilised, yielding 3.15 g (0.63 mmol, 61%) of $SA_{CONH}$ -$PEG_{100}$-NHBoc. The product was characterized by RMN $^1H$ (300 MHz ; $CDCl_3$ ; ppm) : δ : 0.87 (t; J=7.2 Hz ; 3H) ; 1.24 (m ; 28H) ; 1.44 (s ; 9H) ; 1.67 (quin ; 2H) ; 2.42 (t ; J=7.5 Hz ; 2H) ; 3.3 (t ; J=5.0 Hz ; 2H) ; 3.4 (t ; J=5.0 Hz ; 2H) ; 3.48-3.8 (m ; 360H) ; 3.87 (t; J=5.0 Hz ; 2H) ; 7.79 (bs ; 1 H).

*Synthesis of $SA_{CONH}$ -$PEG_{100}$-$NH_3^+TFA^-$*

An amount of 1.61 g (0.3 mmol) of $SA_{CONH}$-$PEG_{100}$-NHBoc and 6.32 g (55 mmol) of trifluoroacetic acid (TFA) were added to 10 mL of DCM until complete dissolution. The mixture was stirred for one hour at room temperature (about 20°C). Solvent and TFA were then evaporated by co-evaporation with toluene under reduced pressure. Precipitation was obtained upon addition of cold diethyl ether. The precipitate was filtered, dissolved in water, extensively dialysed against water (MWCO 1 kDa) and lyophilised, yielding 1.37 g (0.26 mmol, 85%) of $SA_{CONH}$ -$PEG_{100}$-$NH_3^+TFA^-$. The product was characterized by RMN $^1H$ (300 MHz ; $CDCl_3$ ; ppm) : δ : 0.87 (t ; J=7.2 Hz ; 3H) ; 1.24 (m ; 28H) ; 1.60 (quin ; 2H) ; 2.15 (t ; J=7.5 Hz ; 2H) ; 3.17 (bt ; 2H) ; 3.4 (m ; 4H) ; 3.48-3,8 (m ; 360H) ; 3.87 (t; J=5.0 Hz ; 2H) ; 6.14 (bs ; 1H) ; 7.9 (bs ; 2H).

*Synthesis of $SA_{CONH}$ -$PEG_{100}$-mal*

0.1 g of $SA_{CONH}$PEG100-$NH_3^+TFA^-$ (0,02 mmol) and 5 μL of DIEA (2 eq. ; 0,04 mmol) were dissolved in dichloromethane (2 mL) under argon. After 5 minutes under stirring, 20 mg of SMCC (0,06 mmol ; 3 eq) were added. After 1 h of reaction, solvent was evaporated, and the product precipitated in ether to yield after filtration about 0.1 g of $SA_{CONH}$ -$PEG_{100}$-mal as a white powder. CCM ($CH_2Cl_2$ / MeOH 9/1): Rf = 0,25. RMN $^1H$ (300 MHz ; $CDCl_3$) : δ : 0,88 (t ; C**$H_3$**-$CH_2$) ; 1,25 (m ; C**$H_2$** stearate) ; 1,50 (m; C**$H_2$**, cyclohexane); 1,60 (m ; C**$H_2$**-$CH_2$-CONH) ; 1,9 (m ; cyclo-CH-$CH_2$-) ; 2,20 (t ; C$H_2$-CONH) ; 3,42 (m ; C**$H_2$**-NHCO) ; 3,45 (m ; C**$H_2$**-Mal) ; 3,48-3,8 (m ; xC**$H_2$**(PEG) ; C**$H_2$**-NHCO) ; 6,11 (bt; NH) ; 6,70 (s ; HC=CH maleimide).

6.2. Formulation of F120-maleimide nanoparticles

F120-maleimide nanoparticles were then achieved using exactly the same process as described previously and the composition of Table 24. The nanoparticles were purified by dialysis for 3 hours at room temperature (about 20°C) (20°C) against 1000 times their volume in 1X PBS (12-14,000 Da MW cut off membranes, ZelluTrans) before coupling with the protein.

Table 24: Composition of the F120-maleimide emulsions

| | Compound | Provider | Quantity (mg) |
|---|---|---|---|
| Lipid phase | refined soybean oil | Croda Uniqema, France | 150 |
| | Suppocire NC™ | Gatefosse, France | 450 |
| | Lipoid S75™ | Lipoid, Germany | 45 |
| Aqueous phase | Myrjs40™ | Croda Uniqema, France | 212.5 |
| | $SA_{CONH}$ -$PEG_{100}$-mal | Synthetized as described | 2.5 |
| | aqueous medium: 1X PBS or 154 mM NaCl | | qsp 2 ml 5 |

6.3. Coupling of PDGF-BB on F120-maleimide nanoparticles

PDGF-BB (Life Technologies, Saint-Aubin, France) (100 μg) was suspended in 300 μL of 1X PBS. To this solution were added successively 1.7 mg of $NaHCO_3$ (in 20 μL water), 3 mg of EDTA (in 2 μL water), and 40 μg of iminothiolane (in 10 μL water). The solution was mixed at ambient temperature for 2 hours.

For quantification purpose, the protein was fluorescently labelled with fluorescein. To this goal, NHS-fluorescein (Life Technologies, Saint-Aubin, France) (20 μg in 2μL DMSO) was added to the PDGF-BB solution which was mixed for 30 minutes. The mixture was then purified by size exclusion gel chromatography (PD10 columns, GE Healthcare).

To 90 μg of purified protein suspended in 1 mL of 1X PBS at 4°C were added 322 μL of F120-maleimide emulsion (at 100 mg/mL of lipids) (ratio of 60/1 protein/particle). The solution was stirred overnight, then 2 μl of mercapto-ethanol (1% w/v in water) were added and mixing was continued for 2 hours. The F120-PDGF-BB nanoparticles were

then purified by size exclusion gel chromatography (Superdex 200 gel) and fractions of 500 μL were collected. nanoparticle fractions eluted between 1 and 5 mL, whereas the free protein eluted between 9 and 18 mL. When coupling fluorescein-labelled PDGF-BB, relative quantification of the 2 elution bands by fluorescence analysis (Tecan microplaque analyser, analysis of the eluted fractions) indicated coupling yield of 76%. Therefore, F120-PDGF-BB nanoparticles with 48 proteins/particle were achieved (8.2 mg/mL of lipids, 17.6 μg/mL of PDGF-BB). Finally, the emulsions were filtered through a 0.45 μm Millipore membrane.

The emulsions were characterized by their size and polydispersity index, using Dynamic Light Scattering (Zeta Sizer Nano ZS, Malvern Instrument). For this purpose, the emulsions were diluted to achieve 2 mg/ml of components of the nanoparticles (ie: combination of oil + Suppocire + Lipoid + Myrjs40) in sterile PBS 0.1X and were transferred in Zeta Sizer Nano cells (Malvern Instrument) before each measurement, performed in triplicate.

Results are described in Table 25.

Table 25. Nanoparticle diameter, PdI and zeta potential of the prepared emulsion.

| Emulsion | Nanoparticles concentration (mg/mL) | Nanoparticle diameter (nm) | Polydispersity Index (PdI) | Zeta potential (mV) |
|---|---|---|---|---|
| F120-PDGF-BB | 35.5 | 122 ± 1 | 0.11 ± 0.01 | -5.7 ± 3.1 |

6.4. (Collagen / nanoparticles comprising growth factor) material

F120-PDGF-BB nanoparticles were loaded in the collagen gel (Type I collagen from equine Achilles tendon, prepared at 1% w/v in acetic acid pH 3, Euroresearch) at a final weight ratio of dry collagen/nanoparticles 9.5/1 and lyophilized according to procedure described in .2. Finally, a lyophilized white material was obtained, with composition of 0.1 μg of PDGF and 1.05 mg of nanoparticles for 10 mg of dry collagen.

**Example 7: SiRNA** nanoparticles **loaded in collagen material**

7.1. Preparation of SiRNA nanoparticles

Two different cationic nanoparticles were prepared for complexation with SiRNA, then loading in collagen material. For this purpose, two cationic surfactants, DOTAP and a hydrophobically modified chitosan (HM-CS), were used.

7.1.1 Synthesis of the HM-CS surfactant

500 mg of chitosan (Mw = 86,000 g/mol, % of acetylation = 15%, from Sigma-Aldrich), was dissolved in 25 mL of 0.2 M acetic acid overnight, then 10 mL of EtOH was added before the addition of lauryl (C12) aldehyde (11.5 mg, 2.4 eq., Sigma-Aldrich) in 5 mL EtOH. After addition of an excess of sodium cyanohydroborate, the mixture was stirred overnight at room temperature (about 20°C) and the alkylated product was precipitated by the addition of NaOH 1 M. The product was purified by washing several times with EtOH/water (increasing ratio from 70:30 to 100:0).

The degree of substitution of the polysaccharide by the C12 groups was characterized by 1 H NMR using a AMX400 Bruker NMR at 80°C after dissolving HM-CS surfactant in 0.2 M $CD_3COOD$ at a concentration of 5 mg/mL.

The degree of substitution of the polysaccharide by the C12 groups was calculated as the ratio of $-CH_3$ hydrogen of alkyl chain (H7) (~0.8 ppm) to H1 (~4-4.8ppm) of chitosan backbone. It was found equals to 0.022.

7.1.2 Formulation of cationic nanoparticles

Cationic nanoparticles, the composition of which is described in Table 26 were prepared.

Table 26: Composition of the cationic nanoparticles.

| | Compound | Provider | Quantity (mg) | |
|---|---|---|---|---|
| | | | F50 cationic nanoparticles | F130 cationic nanoparticles |
| Lipid phase | refined soybean oil | Croda Uniqema, France | 22.4 | 22.4 |
| | Suppocire NC™ | Gatefosse, France | 67.1 | 67.1 |
| | DOTAP (1,2-dioleoyl-3-trimethylammoniumpropane). | Avanti Polar Lipids, Alabaster AL, USA | 15 | 17 |
| | Lipoid S75™ | Lipoid, Germany | 2.2 | 1.7 |
| | DiD or Dil | Life Technologies, France | 0.239 (DiD) | 0.187 (Dil) |
| Aqueous phase | Myrjs40™ | Croda Uniqema, France | 43.1 | - |
| | HM-CS surfactant | Synthetized in example 6.1.1 | - | 10 |
| | Glycerol | | 1000 | - |
| | aqueous medium: 154 mM NaCl | | qsp 2 ml | - |
| | aqueous medium: 0.2 M acetic acid pH 4 | | - | qsp 2 ml |

An oily premix was prepared by mixing refined soybean oil, Suppocire NC™, Lipoid S75™, DOTAP (1,2-dioleoyl-3-trimethylammonium-propane) and DiD or Dil (for nanoparticle tracking). After homogeneization at 50°C, the continuous aqueous phase, composed of Myrjs40™, HM-CS surfactant, glycerol and of aqueous medium, was introduced. The vial was placed in a 50°C water bath and the mixture was sonicated for 5 min using a VCX750 ultrasonic processor (power output 190 W, 3-mm probe diameter, Sonics). The obtained emulsions were dialyzed overnight at room temperature (about 20°C) (20°C) against 1000 times their volume in the appropriate aqueous buffer (1 X PBS for F50 cationic nanoparticles, 0.1 M acetate buffer pH 5.1 for F130 cationic nanoparticles) using 12-14,000 Da MW cut off membranes (ZelluTrans) for F50 cationic nanoparticles, and 300,000 Da MW cut off membranes (SpectraPor) for F130 cationic nanoparticles. Finally, the emulsions were filtered through a 0.22 μm Millipore membrane (F50 cationic nanoparticles) or through a 0.45 μm Millipore cellulose membrane (F130 cationic nanoparticles).

The emulsions were characterized by their size and polydispersity index, using Dynamic Light Scattering (Zeta Sizer Nano ZS, Malvern Instrument). For this purpose, 20 μL of emulsions were diluted in 980 μL of NaCl 10 mM and were transferred in Zeta Sizer Nano cells (Malvern Instrument) before each measurement, performed in triplicate.

Results are described in Table 27.

Table 27. Nanoparticle diameter, PdI and zeta potential of the prepared cationic emulsions

| Emulsion | Nanoparticles concentration (mg/mL) | Nanoparticle diameter (nm) | Polydispersity Index (PdI) | Zeta potential (mV) |
|---|---|---|---|---|
| F50 cationic nanoparticles | 32 | 68 ± 1 | 0.14 ± 0.02 | +12.9 ± 0.6 |
| F130 cationic nanoparticles | 58 | 129 ± 1 | 0.12 ±0.01 | +48.1 ± 1.5 |

7.1.3 Complexation with SiRNA

siRNA complexation was performed by adding 30 pmol siRNA (All Star negative control SiRNA, Qiagen, France) to an aqueous solution of F50 or F130 cationic nanoemulsions with N/P ratios (N:nitrogen (+) to P, phosphate (-) ratio) of 16:1. The final siRNA concentration was 20 μg/mL, and was 6.6 mg/mL for F50 cationic nanoparticles and 1.64 mg/mL for F130 cationic nanoparticles, which present a higher nitrogen concentration and surface density thanks to the mixture

of DOTAP and HM-CS surfactants.

After 30 minutes incubation, the nanoparticle-siRNA solutions were analysed by electrophoresis using a 4% agarose gel to check that all the siRNA was efficiently bound to the nanoparticle surface. Indeed, we observe in Figure 22 that in comparison to free siRNA used as control that migrated on the gel, no free SiRNA migrated when the siRNA was bound to the cationic nanoparticles at N:P ratio of above 4:1, demonstrating the good complexation between the nanoparticles and the siRNA for both the F50-SiRNA and F130-siRNA conjugates.

The nanoparticles/SiRNA conjugates were then characterized by their size and polydispersity index, using Dynamic Light Scattering (Zeta Sizer Nano ZS, Malvern Instrument). For this purpose, the 20 μL of the conjugate solution were diluted in 980 μL of NaCl 10 mM and were transferred in Zeta Sizer Nano cells (Malvern Instrument) before each measurement, performed in triplicate.

Results are described in Table 28.

Table 28. Nanoparticle/siRNA complex diameter, PdI and zeta potential of the prepared emulsions

| Emulsion | Nanoparticle/siRNA complex diameter (nm) | Polydispersity Index (PdI) | Zeta potential (mV) |
|---|---|---|---|
| F50-siRNA | 77±0.5 | 0.121 ± 0.003 | +13.9 ± 0.7 |
| F130-siRNA | 142±1 | 0.189 ± 0.02 | +44.5 ± 1.5 |

7.2 (Collagen / nanoparticles comprising siRNA) material

F50-siRNA and F130-SiRNA nanoparticles prepared with a 16:1 N:P ratio were loaded in the collagen gel (Type I collagen from equine Achilles tendon, prepared at 1% w/v in acetic acid pH 3, Euroresearch) at a final weight ratio of 1 μg of SiRNA (either labelled with AF488 for F50 cationic nanoparticles, or with AF647 for F130 cationic nanoparticles, All stars negative Control, Qiagen, France) per 20 mg of dried collagen (ie 2 g of collagen gel at 1% w/v in acetic acid pH 3). After gentle mixing of the collagen gel and the F50-siRNA or F130-SiRNA nanoparticles for 1 h, the gels were lyophilized in a well of a 12-well plate according to the procedure described previously in example 4.2. Finally, a lyophilized material was obtained, with composition of 20 mg of dry collagen, 1 μg of siRNA, and 0.351 mg of F50 cationic nanoparticles for (collagen/F50-siRNA) material, or 0.084 mg of F130 cationic nanoparticles for (collagen/F130-siRNA) material.

7.3 Kinetics of release from (Collagen / nanoparticles comprising siRNA) material

The release of both the F50 or F130 cationic nanoparticles, and the siRNA, was evaluated when the (Collagen / nanoparticles comprising siRNA) materials were incubated in PBS at 37°C. For this purpose, fluorescence quantitation was performed using the fluorescent DiD dye loaded in the core of the cationic F50 and Dil dye loaded in F130 nanoparticles (fluorescence measurements with an excitation at 630 nm, and emission recorded at 670 nm for DiD and excitation at 520 and emission at 565 for Dil) and AF488 or AF647 which are the fluorescent labels on siRNA sequence (fluorescence measurements with an excitation at 490 nm, and emission recorded at 520 nm for AF488 and an excitation at 630 nm, and emission recorded at 670 nm for AF647).

The materials (about 20 mg) were then incubated in 2 mL of 1X PBS at 37°C, and siRNA and nanoparticle release from the collagen material was evaluated by fluorescence measurements in the 1X PBS medium at different time points and quantified using a calibration curve. Comparative measurements for siRNA release were made with a siRNA-collagen material composed of 20 mg of collagen and 1 μg of siRNA, prepared by mixing directly 1 μg of siRNA in 2 g of collagen gel, then lyophilization of the material as previously described. Comparative measurements for nanoparticle release were made with a F50-collagen material (composed of 20 mg of collagen and 0.35 mg of F50 cationic nanoparticles) or a F130-collagen material (composed of 20 mg of collagen and 0.084 mg of F130 cationic nanoparticles).

Results are described in Tables 29 and 30.

Table 29. % of nanoparticles released when incubating 20 mg of material in 2 mL of PBS at 37°C.

| Time (hours) | % of nanoparticles released from the (collagen-F50) material | % of nanoparti cles released from the (collagen /F50-siRNA) material | % of nanopartic les released from the (collagen/ F130) material | % of nanopartic les released from the (collagen/ F130-siRNA) material |
|---|---|---|---|---|
| 2 | <5 | <5 | 5±5 | 9±5 |
| 4 | <5 | <5 | 9±5 | 13±5 |

(continued)

| Time (hours) | % of nanoparticles released from the (collagen-F50) material | % of nanoparti cles released from the (collagen /F50-siRNA) material | % of nanopartic les released from the (collagen/ F130) material | % of nanopartic les released from the (collagen/ F130-siRNA) material |
|---|---|---|---|---|
| 6 | <5 | <5 | 17±5 | 13±5 |
| 24 | <5 | <5 | 22±5 | 17±5 |
| 48 | <5 | <5 | 26±5 | 22±5 |
| 72 | <5 | <5 | 30±5 | 31±5 |
| 96 | <5 | <5 | 35±5 | 35±5 |

Table 30. % of SiRNA released when incubating 20 mg of material in 2 mL of PBS at 37°C.

| Time (hours) | % of siRNA released from the (collagen-SiRNA) material | % of siRNA released from the (collagen/F50-siRNA) material | % of siRNA released from the (collagen/F130 - siRNA) material |
|---|---|---|---|
| 2 | 15 ± 5 | 16 ± 5 | < 5 |
| 4 | 27 ± 5 | 32 ± 5 | 13 ± 5 |
| 6 | 38 ± 5 | 42 ± 5 | 27 ± 5 |
| 24 | 58 ± 5 | 63 ± 5 | 27 ± 5 |
| 48 | 77 ± 5 | 82 ± 5 | 40 ± 5 |
| 72 | 89 ± 5 | 94 ± 5 | 40 ± 5 |
| 96 | 97 ± 5 | 101 ± 5 | 40 ± 5 |

The results show that the F50 cationic nanoparticles were well retained in the gel matrix, whereas the F130 nanoparticles, despite their larger size and density of charges were partially leaking out of the material, however in a moderate way since 65% of the nanoparticles were still retained in the hydrogels 4 days after incubation (Table 29). The SiRNA complexation did not modify the release profiles of the nanoparticles.

SiRNA release form the (collagen-SiRNA) material occurred in 96 hours. Complexation of SiRNA with F50 nanoparticles before gel loading did not significantly modified SiRNA release; on the contrary, complexation of SiRNA with F130 nanoparticles before gel loading prevented SiRNA to be released from the collagen gel (Table 30): 40 ± 5% of SiRNA was released from the (collagen/F130 -siRNA) hydrogel after 96 h incubation at 37°C in 1X PBS, correlating with 35 ± 5 % released of F130 nanoparticles during the same period.

## Claims

**1.** Material comprising:

- a polymer capable of forming a hydrogel, and
- nanoparticles comprising:
- a solubilising lipid comprising at least one fatty acid glyceride,
- an amphiphilic lipid, and
- a co-surfactant comprising at least one poly(ethylene oxide) chain or a chitosan chain.

**2.** Material according to claim 1, wherein:

- the polymer capable of forming a hydrogel is chosen from chitosan, chitosanpolyethylene glycol copolymers, carboxymethyl cellulose, carboxymethyl cellulosepolyethylene glycol copolymers, collagen and hyaluronic acid, and/or
- the amphiphilic lipid is a phospholipid, and/or

- the solubilising lipid is a mixture of glycerides of saturated fatty acids comprising at least 10 % by weight of C12 fatty acids, at least 5 % by weight of C14 fatty acids, at least 5 % by weight of C16 fatty acids and at least 5 % by weight of C18 fatty acids.

**3.** Material according to anyone of claims 1 to 2, wherein the nanoparticles comprise a therapeutic agent.

**4.** Material according to claim 3, wherein the therapeutic agent is therapeutic agent chosen from hormones, vitamins, growth factors, cell adhesion factors, cell migration factors, chemotherapeutic agents, antibiotics, antimicrobial agents, saccharides, and nucleotide sequences, such as DNA, mRNA or nucleotide sequences able to modulate endogenous mechanisms of RNA interference, for example siRNA.

**5.** Material according to claim 3, wherein the therapeutic agent is chosen from chemical compounds capable of promoting wound healing, growth factors, antibiotics, antimicrobial agents, saccharides, and nucleotide sequences, such as DNA, mRNA or nucleotide sequences able to modulate endogenous mechanisms of RNA interference, for example siRNA.

**6.** Material according to claim 3, wherein the therapeutic agent is a nucleotide sequence able to modulate endogenous mechanisms of RNA interference, such as:

- a short or small interfering RNA - siRNA,
- a locked nucleic acid - LNA,
- a synthetic microRNA mimic called MicroRNA or miRNA.

**7.** Material according to claim 3, wherein the therapeutic agent is an antigen or a nucleic acid molecule encoding one or several protein antigens.

**8.** Material according to anyone of claims 1 to 7, wherein the weight of polymer capable of forming a hydrogel compared to the weight of the nanoparticles is from 0.05/1 to 1000/1.

**9.** Material according to anyone of claims 1 to 8, in the form of a hydrogel.

**10.** Material according to anyone of claims 1 to 8, in a dry form.

**11.** Material according to anyone of claims 1 to 10, wherein the nanoparticles are homogeneously distributed within the material.

**12.** Process for the preparation of a material according to anyone of claims 1 to 11, comprising the steps consisting in:

A1) providing a nanoemulsion comprising a continuous aqueous phase and nanoparticles comprising an amphiphilic lipid, a solubilising lipid comprising at least one fatty acid glyceride and a co-surfactant comprising at least one poly(ethylene oxide) chain or a chitosan chain,
A2) mixing the nanoemulsion obtained in step A1) with a polymer capable of forming a hydrogel to obtain the material in hydrogel form,
and optionally drying the material in hydrogel form obtained at the end of step A2) to obtain the material in a dry form.

**13.** Process according to claim 12, wherein the nanoemulsion of step A1) is obtained by a process comprising the following steps:

A1a) preparing an oily phase comprising the solubilising lipid and the amphiphilic lipid,
A1b) preparing an aqueous phase comprising the co-surfactant;
A1c) dispersing the oily phase in the aqueous phase under the action of sufficient shear to form a nanoemulsion,
A1d) optionally recovering the nanoemulsion thus formed.

**14.** Process for the preparation of a material according to anyone of claims 1 to 11, comprising the steps consisting in:

B1) preparing an oily phase comprising a solubilising lipid comprising at least one fatty acid glyceride and an amphiphilic lipid,

B2) preparing an aqueous phase comprising a co-surfactant comprising at least one poly(ethylene oxide) chain or a chitosan chain and a polymer capable of forming a hydrogel;
B3) dispersing the oily phase in the aqueous phase under the action of sufficient shear to obtain the material in hydrogel form,
and optionally drying the material in hydrogel form obtained at the end of step B3) to obtain the material in a dry form.

**15.** Use of the material according to anyone of claims 1 to 11 for wound healing dressings, cell culture materials, tissue engineering, implants, patches, coatings, sensors, in diagnostic, pharmaceutical, or cosmetic industry.

**16.** Material according to claim 3, for its use for the prevention and/or the treatment of a disease.

**17.** Use of the material according to claim 4 as a material for cell culture.

**18.** Use of the material according to claim 5 as a material of a dressing, of an implant or of an implant coating.

**19.** Use of the material according to claim 6 as transfecting agent.

**20.** Material according to claim 7, for its use to induce an immune response against the antigen present in the material, or against the antigen encoded by the nucleic acid molecule present in the material.

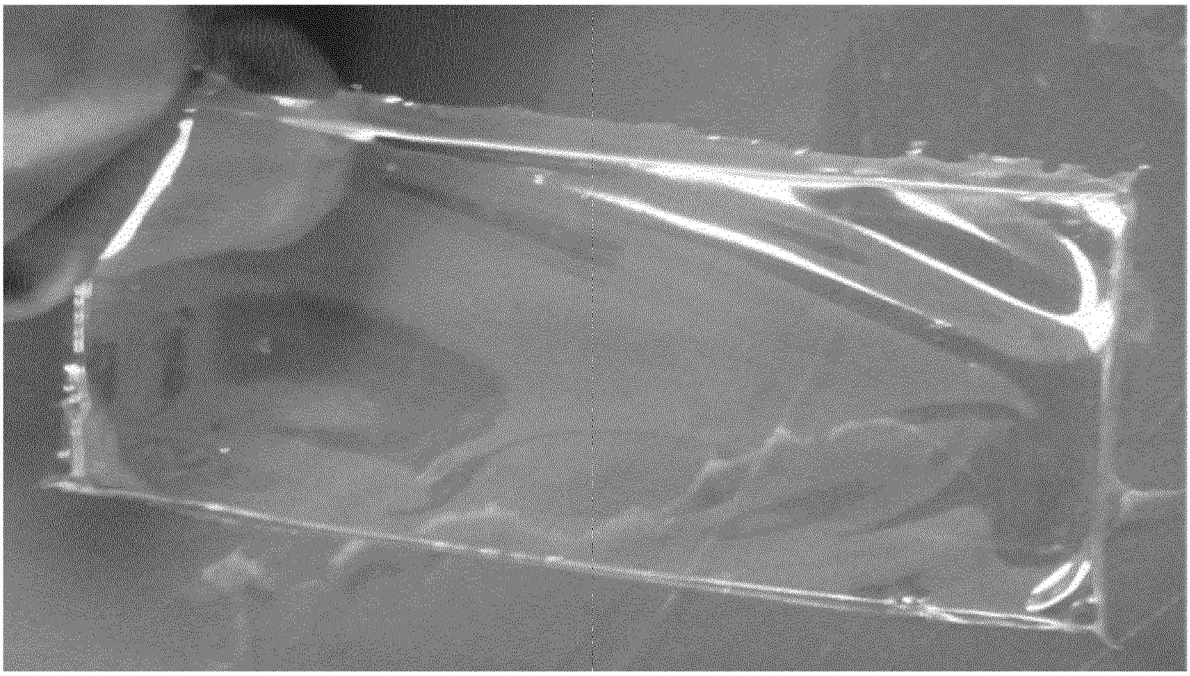

Fig. 1

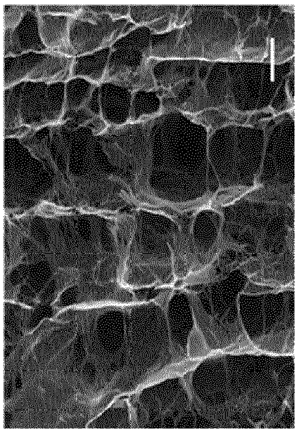
Fig. 2

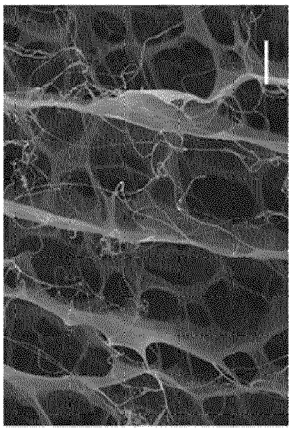
Fig. 3

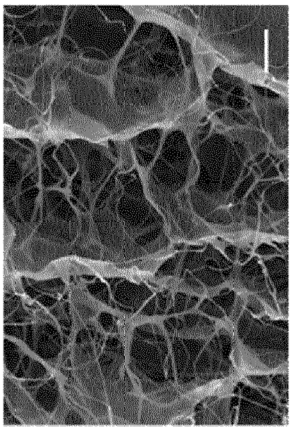
Fig. 4

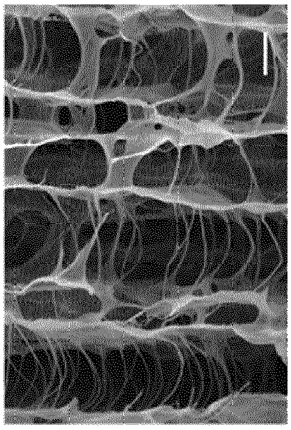
Fig. 5

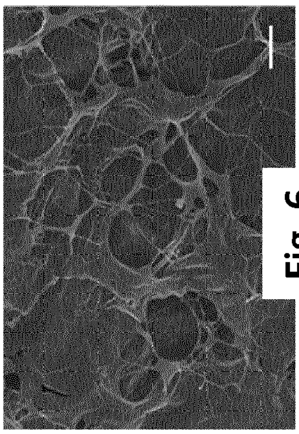
Fig. 6

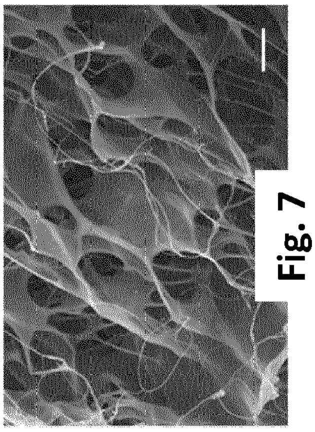
Fig. 7

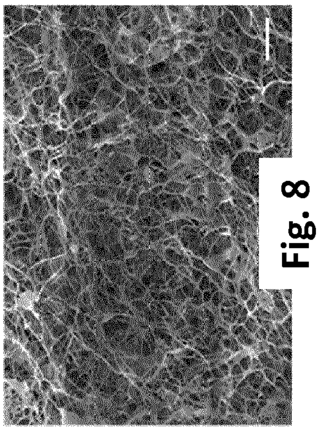
Fig. 8

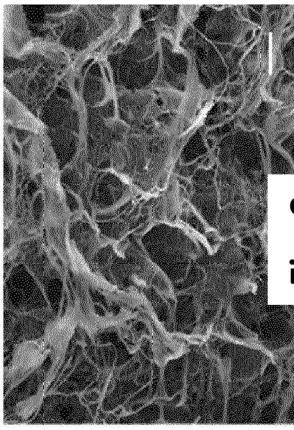
Fig. 9

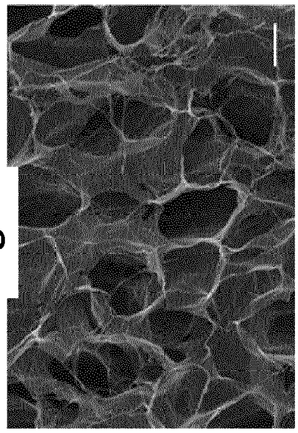
Fig. 10

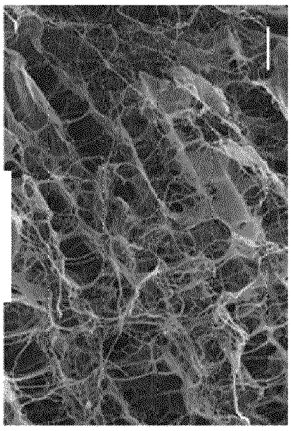
Fig. 11

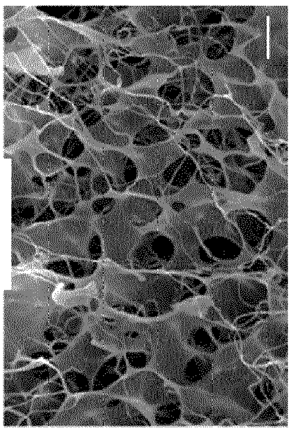
Fig. 12

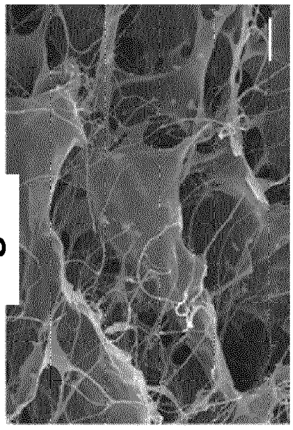
Fig. 13

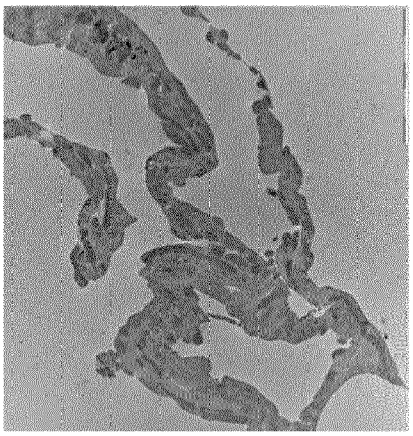
Fig. 14

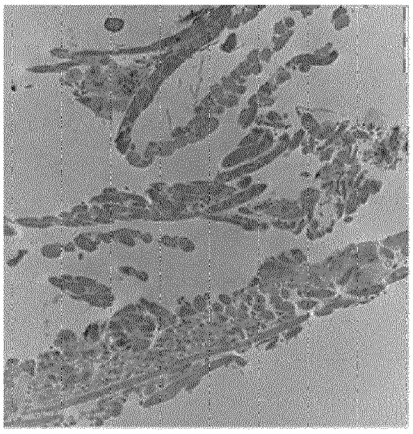
Fig. 15

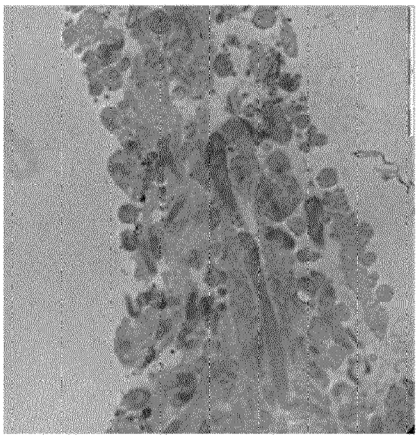
Fig. 16

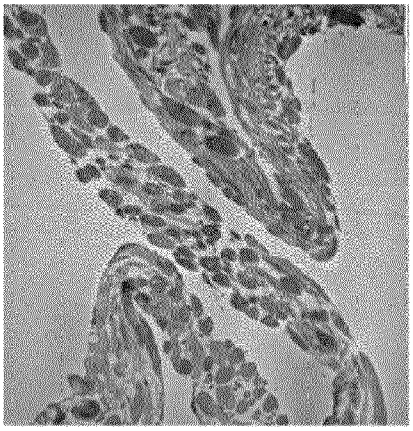
Fig. 17

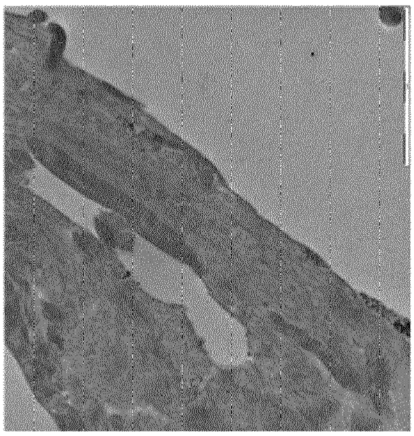
Fig. 18

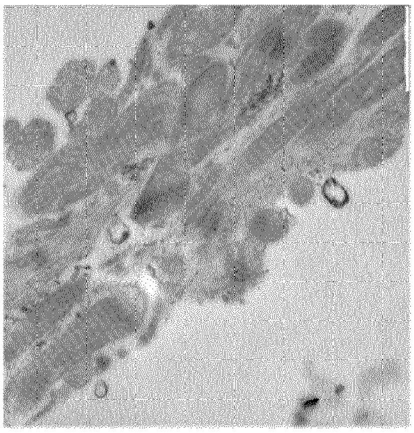
Fig. 19

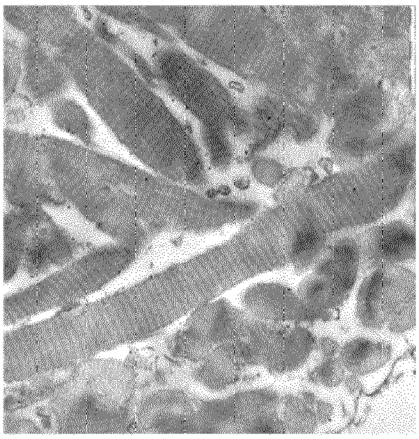
Fig. 20

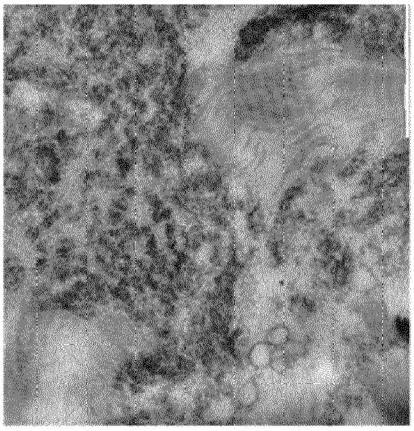
Fig. 21

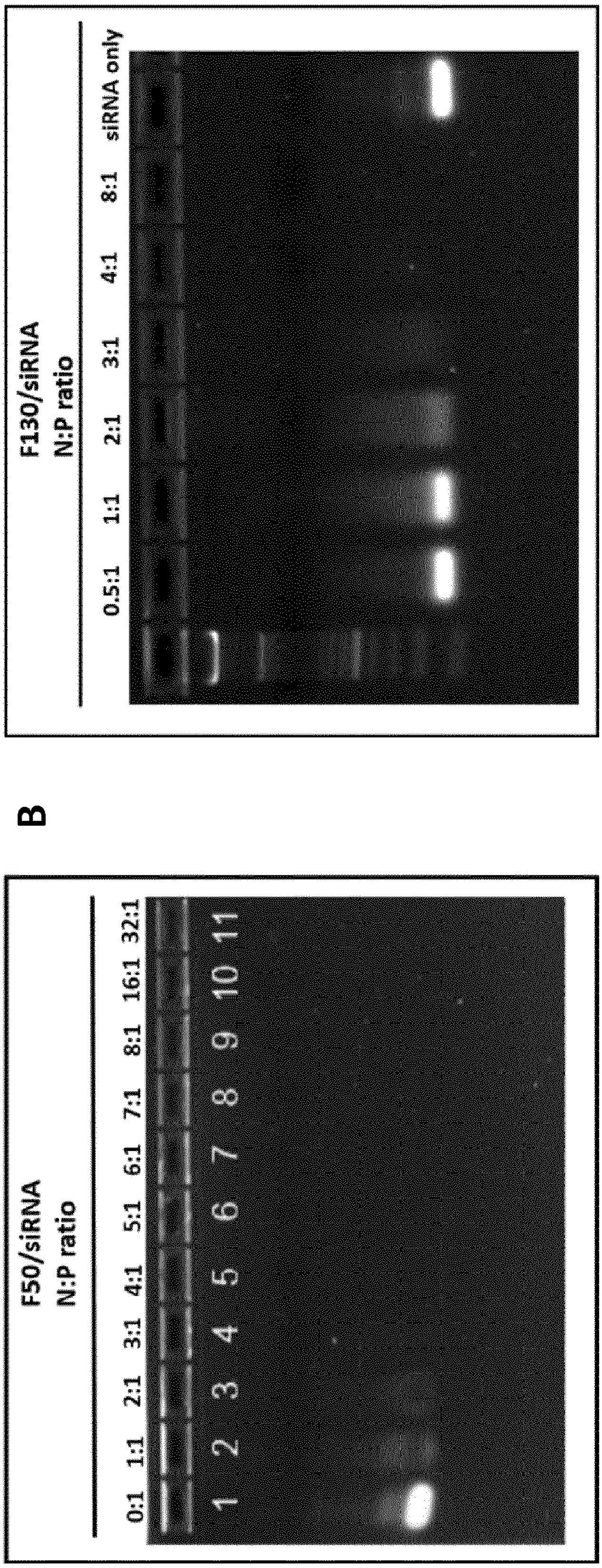

A
F50/siRNA
N:P ratio
0:1
1:1
2:1
3:1
4:1
5:1
6:1
7:1
8:1
16:1
32:1
1
2
3
4
5
6
7
8
9
10
11
B
F130/siRNA
N:P ratio
0.5:1
1:1
2:1
3:1
4:1
8:1
siRNA only


Fig. 22

Europäisches Patentamt
European Patent Office
Office européen des brevets

# EUROPEAN SEARCH REPORT

Application Number
EP 15 30 5896

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PREGO C ET AL: "Chitosan-PEG nanocapsules as new carriers for oral peptide delivery", JOURNAL OF CONTROLLED RELEASE, ELSEVIER, AMSTERDAM, NL, vol. 111, no. 3, 10 April 2006 (2006-04-10), pages 299-308, XP024957459, ISSN: 0168-3659, DOI: 10.1016/J.JCONREL.2005.12.015 [retrieved on 2006-04-10] * page 300, column 2, paragraph 4 - page 301, column 1, paragraph 5 * ----- | 1-4,11, 14-16 | INV. A61L27/48 A61L27/52 A61L27/54 A61L31/12 A61L31/14 A61L31/16 A61K47/10 A61K47/14 A61K47/36 A61K9/107 |
| X | CN 103 371 973 A (UNIV FUDAN) 30 October 2013 (2013-10-30) * example 1 * ----- | 1-4,9, 11-16 | |
| X | GARCIA-FUENTES M ET AL: "A comparative study of the potential of solid triglyceride nanostructures coated with chitosan or poly(ethylene glycol) as carriers for oral calcitonin delivery", EUROPEAN JOURNAL OF PHARMACEUTICAL SCIENCES, ELSEVIER, AMSTERDAM, NL, vol. 25, no. 1, 1 May 2005 (2005-05-01), pages 133-143, XP027803644, ISSN: 0928-0987 [retrieved on 2005-05-01] * page 134, column 2, paragraph 2 - page 135, column 2, paragraph 6 * ----- | 1-4,8, 10,11, 14-17 | TECHNICAL FIELDS SEARCHED (IPC): A61L A61K C12N |
| A | EP 2 460 516 A2 (UNIV PAIS VASCO [ES]) 6 June 2012 (2012-06-06) * paragraphs [0001], [0009] - [0013], [0020], [0031], [0039] * * example 19 * * claims 1-7 * ----- | 1-4,8-17 | |

2

~~The present search report has been drawn up for all claims~~

| Place of search | Date of completion of the search | Examiner |
|---|---|---|
| The Hague | 27 November 2015 | Cadamuro, Sergio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 15 30 5896

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1, 3, 8-17(completely); 2, 4(partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number

EP 15 30 5896

The Search Division considers that the present European patent application does not comply with the requirements of unity of invention and relates to several inventions or groups of inventions, namely:

1. claims: 1, 3, 8-17(completely); 2, 4(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the polymer capable of forming a hydrogel is chitosan (or chitosan-PEG copolymer); and the therapeutic agent is a hormone.

---

2. claim: 2(partially)

Material comprising:- a polymer capable of forming a hydrogel, and - nanoparticles comprising:- a solubilising lipid comprising at least one fatty acid glyceride, - an amphiphilic lipid, and - a co-surfactant comprising at least one poly(ethylene oxide) chain or a chitosan chain. Wherein the polymer capable of forming a hydrogel is CMC (or CMC-PEG copolymer).

---

3. claim: 2(partially)

Material comprising:- a polymer capable of forming a hydrogel, and - nanoparticles comprising:- a solubilising lipid comprising at least one fatty acid glyceride, - an amphiphilic lipid, and - a co-surfactant comprising at least one poly(ethylene oxide) chain or a chitosan chain. Wherein the polymer capable of forming a hydrogel is collagen.

---

4. claim: 2(partially)

Material comprising:- a polymer capable of forming a hydrogel, and - nanoparticles comprising:- a solubilising lipid comprising at least one fatty acid glyceride, - an amphiphilic lipid, and - a co-surfactant comprising at least one poly(ethylene oxide) chain or a chitosan chain. Wherein the polymer capable of forming a hydrogel is hyaluronic acid.

---

5. claim: 4(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a

Europäisches Patentamt
European Patent Office
Office européen des brevets

# LACK OF UNITY OF INVENTION SHEET B

Application Number
EP 15 30 5896

The Search Division considers that the present European patent application does not comply with the requirements of unity of invention and relates to several inventions or groups of inventions, namely:

therapeutic agent. Wherein the therapeutic agent is a vitamin.

---

6. claims: 4, 5(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is a growth factor.

---

7. claims: 4, 5(partially)

Material comprising:- a polymer capable of forming a hydrogel, and - nanoparticles comprising:- a solubilising lipid comprising at least one fatty acid glyceride, - an amphiphilic lipid, and - a co-surfactant comprising at least one poly(ethylene oxide) chain or a chitosan chain. Wherein the therapeutic agent is a cell adhesion factor.

---

8. claim: 4(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is a cell migration factor.

---

9. claim: 4(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is a chemotherapeutic agent.

---

10. claim: 4(partially)

Material comprising:- a polymer capable of forming a hydrogel, and - nanoparticles comprising:- a solubilising

Europäisches Patentamt
European Patent Office
Office européen des brevets

## LACK OF UNITY OF INVENTION SHEET B

Application Number
EP 15 30 5896

The Search Division considers that the present European patent application does not comply with the requirements of unity of invention and relates to several inventions or groups of inventions, namely:

lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is an antibiotic.

---

11. claims: 4, 5(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is an antimicrobial agent.

---

12. claims: 4, 5(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is a saccharide.

---

13. claims: 6, 19(completely); 4, 5, 7(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is a nucleotide sequence.

---

14. claims: 18(completely); 5(partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is a chemical agent capable of promoting wound healing.

---

Europäisches Patentamt
European Patent Office
Office européen des brevets

# LACK OF UNITY OF INVENTION SHEET B

Application Number
EP 15 30 5896

The Search Division considers that the present European patent application does not comply with the requirements of unity of invention and relates to several inventions or groups of inventions, namely:

15. claims: 7, 20(all partially)

Material comprising:- a polymer capable of forming a hydrogel, and - nanoparticles comprising:- a solubilising lipid comprising at least one fatty acid glyceride, - an amphiphilic lipid, - a co-surfactant comprising at least one poly(ethylene oxide) chain or a chitosan chain and - a therapeutic agent. Wherein the therapeutic agent is an antigen.

---

## ANNEX TO THE EUROPEAN SEARCH REPORT ON EUROPEAN PATENT APPLICATION NO. EP 15 30 5896

This annex lists the patent family members relating to the patent documents cited in the above-mentioned European search report. The members are as contained in the European Patent Office EDP file on
The European Patent Office is in no way liable for these particulars which are merely given for the purpose of information.

27-11-2015

| Patent document cited in search report | Publication date | Patent family member(s) | Publication date |
|---|---|---|---|
| CN 103371973 A | 30-10-2013 | NONE | |
| EP 2460516 A2 | 06-06-2012 | EP 2460516 A2 | 06-06-2012 |
| | | ES 2351756 A1 | 18-02-2011 |
| | | US 2012183589 A1 | 19-07-2012 |
| | | WO 2011015701 A2 | 10-02-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010018223 A
- WO 2011101602 A
- WO 2013144369 A
- WO 2014131809 A
- WO 2014032953 A

### Non-patent literature cited in the description


- **C. J. DOILLON ; C. F. WHYNE ; S. NBRANDWEIN ; F. H. SILVER.** Collagen-based wound dressings: control of the pore structure and morphology. *J. Biomed. Mater. Res.,* 1986, vol. 20, 1219-1228
- **MERINO et al.** *ACSNano,* 2015, vol. 9 (5), 4686-4697
- **W.-C. LIN ; W. FAN ; A. MARCELLAN ; D. HOURDET ; C. CRETON.** Large Strain and Fracture Properties of Poly(dimethylacrylamide)/Silica Hybrid Hydrogels. *Macromolecules,* 2010, vol. 43, 2554-2563